# EUROPEAN PATENT APPLICATION

(11) **EP 4 129 383 A1**
(43) Date of publication of application: **08.02.2023**
(21) Application number: 21779416.3
(22) Date of filing: 30.03.2021
(51) Int. Cl.: A61M 25/06

(54) **INDWELLING NEEDLE**

(30) Priority: 30.03.2020 JP 2020060808
(71) Applicant: Nipro Corporation, Osaka-shi, Osaka 531-8510 (JP)
(72) Inventor: MIYAKE, Takako, Osaka-shi, Osaka 531-8510 (JP); YOKOTA, Hiroki, Osaka-shi, Osaka 531-8510 (JP)
(74) Representative: Novagraaf International SA
(86) International application number: PCT/JP2021/013657
(87) International publication number: WO 2021/201014

(57) **Abstract**

Provided is an indwelling needle that is less likely to cause blood stagnation in the vicinity of an elastic member. The present invention provides an indwelling needle comprising: an inner needle that punctures a living body; an outer needle that is inserted into the living body together with the inner needle as the inner needle penetrates the outer needle and punctures the living body; a main body that is disposed along the axis of the inner needle; and a branch part that branches from the main body. The indwelling needle is provided with: an outer needle hub that has a passage in communication with the interior of the outer needle; an elastic member that is disposed within the outer needle hub, is penetrated by the inner needle, and has a flexible solid part that blocks fluid flow to the proximal end side of the main body when the inner body is removed; and a holder that is attached to the proximal end of the elastic member. The elastic member forms at least part of the passage that allows communication between the interior of the outer needle and the branch part, and has an end surface inclined relative to the axial direction. The outer needle hub has a guide part that is engaged with the elastic member or the holder and guides the rotation direction of the elastic member around the axis to a certain direction.

## Description

### Technical Field

The present invention relates to an indwelling needle including an outer needle that is indwelled by puncturing a living body.

### Background Art

An indwelling needle has been developed as a medical instrument used for infusion such as drip infusion. The indwelling needle includes an inner needle that punctures a blood vessel of a patient, and an outer needle that is penetrated by the inner needle and is inserted into the blood vessel of the patient together with the inner needle as the inner needle punctures the living body. The outer needle is indwelled in the blood vessel of the patient, collecting blood from or giving a drip to the patient via the outer needle. As such an indwelling needle, Patent Literature 1 discloses an indwelling needle in which an outer needle hub is provided with a tube on its lateral side and a rubber stopper (the rubber stopper is also referred to as an elastic member) is provided at a proximal end of the outer needle hub.

### Citation List

### Patent Literature

Patent Literature 1: JP 2004-528127 A

### Summary of Invention

### Technical Problem

In the indwelling needle as described above, since there is a space between the rubber stopper and the tube in the outer needle hub, stagnation can occur in the vicinity of the rubber stopper. For this reason, for example, when the indwelling needle is used for an operation of allowing a liquid (blood) having a coagulation component to flow for a long time, such as artificial dialysis, blood clots may be generated in the vicinity of the rubber stopper.

Therefore, an object of the present invention is to provide an indwelling needle in which stagnation hardly occurs in the vicinity of an elastic member.

### Solution to Problem

In order to achieve the above object, the present invention adopts the following configurations.

That is, an indwelling needle according to one aspect of the present invention includes:
an inner needle that punctures a living body;
an outer needle that is penetrated by the inner needle and is inserted into the living body together with the inner needle as the inner needle punctures the living body;
an outer needle hub including a main body that is arranged along an axis of the inner needle and a branch part that branches from the main body, the outer needle hub having a passage communicating with an inside of the outer needle;
an elastic member that is disposed inside the outer needle hub, is penetrated by the inner needle, and has a soft solid part that blocks a fluid flow to a proximal end side of the main body in a case where the inner needle is removed; and
a holder that is attached to a proximal end of the elastic member, wherein
the elastic member has an end surface that forms at least a part of the passage bringing the inside of the outer needle and the branch part into communication, and that is inclined with respect to a direction of the axis, and
the outer needle hub has a guiding part that is engaged with the elastic member or the holder to guide a direction around the axis of the elastic member in a certain direction.

In this way, the elastic member can be positioned by the guiding part in such a direction around the axis that the inclined surface is directed toward the branch part. The inclined surface of the elastic member can make the passage from a distal end of the outer needle hub to the branch part smoother than a conventional one. As a result, the fluidity of a liquid in the outer needle hub is improved, and the occurrence of stagnation in the vicinity of the elastic member is reduced.

In the present invention, the elastic member has a central part where the inclined end surface is formed, and an outer peripheral part formed on the outer peripheral side of the central part. A peripheral wall part having a proximal end surface facing a distal end surface of the outer peripheral part is formed on an inner surface of the outer needle hub. At least a part of the central part is located inside the peripheral wall part. In this way, a step is less likely to be formed between the elastic member and the peripheral wall part, and it is possible to suppress formation of a space that causes stagnation.

A guided part to be engaged with the guiding part of the present invention may be the inclined surface as the distal end surface of the elastic member, or may be a recess or a protrusion formed on an outer surface of the elastic member. The guided part may be formed on a distal end surface of the holder, or may be a recess or a protrusion formed on an outer surface of the holder. That is, the guiding part may be the peripheral wall part, or may be a protrusion or a recess extending in the axis direction. In addition, the guiding part may be a screw part that defines the direction around the axis in a certain direction. Furthermore, the guiding part is not limited to the inner surface of the outer needle hub, and may be provided on an outer surface of the outer needle hub. The guided part extending to the outer surface of the outer needle hub may be formed in the holder. In addition, an inclined surface having an inclination direction different from that of the inclined surface of the elastic member may be formed on the hub inner surface and the elastic member or the holder, and the direction around the axis of the elastic member may be set to a predetermined direction by overlapping the surfaces.

In the present invention, the end surface of the central part of the elastic member and an end surface of the peripheral wall part are formed to have a long axis and a short axis. In this way, the central part can be easily positioned in the peripheral wall part of the outer needle hub, and the elastic member can be easily defined in the predetermined direction around the axis.

In the present invention, the long axis of the central part of the elastic member is along an axis direction of the branch part. In this way, stagnation of a liquid can be made difficult to occur when the liquid moves from the branch part to the outer needle or from the outer needle to the branch part.

In the present invention, a step between the central part and the outer peripheral part of the elastic member is inclined. In this way, the central part can be easily located in the peripheral wall part, and a fixing force can be improved by increasing a contact area with the peripheral wall part.

In the present invention, a slit formed in the central part of the elastic member is a straight line along the long axis direction of the central part of the elastic member.

In the present invention, the proximal end of the elastic member is externally inserted onto the holder. In this way, when the elastic member is mounted to the outer needle hub, the elastic member is less likely to escape, and stable mounting can be achieved. A part of a proximal end surface of the elastic member may be a plane perpendicular to the axis direction. It is preferable to provide a surface to be engaged with the part of the proximal end surface of the elastic member, on the distal end surface of the holder, because the elastic member can be made less likely to be deformed when the elastic member is moved via the holder.

In the present invention, a part of the proximal end surface of the elastic member is an inclined surface inclined with respect to the axis direction, and the holder has an inclined surface that is engaged with the inclined surface. In this way, relative rotation between the elastic member and the holder can be suppressed.

In the present invention, a recess or a protrusion may be provided on a part of the proximal end surface of the elastic member, and a protrusion or a recess may be provided on the distal end surface of the holder to suppress relative rotation between the elastic member and the holder. In this way, relative rotation between the elastic member and the holder can be suppressed.

In the present invention, the guiding part is the proximal end surface formed in the peripheral wall part and inclined with respect to the axis direction, and the outer peripheral part has the distal end surface that is engaged with the proximal end surface of the peripheral wall part.

In this way, deformation of the elastic member in the proximal end direction is restricted by the holder. When the elastic member is pushed into the outer needle hub, the outer peripheral part and the peripheral wall part come into abutment to generate a guiding action in the direction around the axis in the elastic member. Since the guiding action is generated until the outer peripheral part and the peripheral wall part are engaged with each other, the elastic member is defined in the predetermined direction around the axis.

In the present invention, the guiding part is a rotation preventing mechanism that prevents rotation of the elastic member about the axis.

In the present invention, the holder includes a locking part that restricts relative movement in the axis direction with respect to the outer needle hub.

In the holder, means for restricting the relative movement in the axis direction with respect to the outer needle hub is not limited to the locking part, and the holder and the outer needle hub may be fixed to each other with an adhesive or the like.

In the present invention, the elastic member is compressed radially inward. Such compression prevents liquid leakage from the slit. Note that the compression may be performed by the outer needle hub or by the holder.

In the present invention, the central part is compressed inward by the peripheral wall part.

In the present invention, the holder has an annular step part adjacent to the central part of the elastic member. In this way, the central part of the elastic member that directly receives pressure from the liquid can be made less likely to be deformed toward the proximal end side, and a stagnation space can be made less likely to be generated on the distal end side of the elastic member.

In the present invention, the elastic member has a cylindrical part extending from the outer peripheral part toward the proximal end, and the holder has a small diameter part inserted into the cylindrical part and a large diameter part abutting on a proximal end of the cylindrical part. In this way, the elastic member can be stably mounted to the holder. In addition, the thickness of the holder can be reduced to decrease a necessary external force in pulling out the inner needle.

In the present invention, the peripheral wall part and a distal end part of the holder face each other to compress the elastic member in the axis direction.

In the present invention,
the holder includes an attachment-target part to which the elastic member is attached, and
the elastic member includes, on a proximal end side of the solid part, a cylindrical part whose inner diameter side is supported by the attachment-target part, the cylindrical part having a radial thickness larger than an interval between the attachment-target part and a surface of the outer needle hub radially facing the attachment-target part in a state before being mounted to the outer needle hub.

In this way, a gap between the elastic member and the surface of the outer needle hub on which the elastic member is disposed is sealed by the cylindrical part compressed between the attachment-target part of the holder and the surface of the outer needle hub, so that it is possible to prevent the fluid from flowing to the proximal end side of the main body through between the elastic member and the surface of the outer needle hub on which the elastic member is disposed.

The radial thickness of the cylindrical part is not limited to a case where the thickness is constant in the axis direction, and at least a part of the cylindrical part in the axis direction may have a radial thickness larger than the interval between the attachment-target part and the surface of the outer needle hub radially facing the attachment-target part.

That is, an indwelling needle according to one aspect of the present invention includes:
an inner needle that punctures a living body;
an outer needle that is penetrated by the inner needle and is inserted into the living body together with the inner needle as the inner needle punctures the living body;
an outer needle hub including a main body that is arranged along an axis of the inner needle and a branch part that branches from the main body, the outer needle hub having a passage communicating with an inside of the outer needle; and
an elastic member that is disposed inside the outer needle hub, is penetrated by the inner needle, and has a soft solid part that blocks a fluid flow to a proximal end side of the main body in a case where the inner needle is removed, wherein
the elastic member has an end surface that forms at least a part of the passage bringing the inside of the outer needle and the branch part into communication, and that is inclined with respect to a direction of the axis, and
the outer needle hub has a guiding part that is engaged with the elastic member to guide a direction around the axis of the elastic member in a certain direction.

In this way, the elastic member can be positioned by the guiding part in such a direction around the axis that the inclined surface is directed toward the branch part. The inclined surface of the elastic member can make the passage from a distal end of the outer needle hub to the branch part smoother than a conventional one. As a result, the fluidity of a liquid in the outer needle hub is improved, and the occurrence of stagnation in the vicinity of the elastic member is reduced.

That is, an indwelling needle according to one aspect of the present invention includes:
an inner needle that punctures a living body;
an outer needle that is penetrated by the inner needle and is inserted into the living body together with the inner needle as the inner needle punctures the living body;
an outer needle hub including a main body that is arranged along an axis of the inner needle and a branch part that branches from the main body, the outer needle hub having a passage communicating with an inside of the outer needle; and
an elastic member that is disposed inside the outer needle hub, is penetrated by the inner needle, and has a soft solid part that blocks a fluid flow to a proximal end side of the main body in a case where the inner needle is removed, wherein
the elastic member has a central part having an end surface that forms at least a part of the passage bringing the inside of the outer needle and the branch part into communication, and that is inclined with respect to a direction of the axis, and an outer peripheral part, and
a peripheral wall part having a proximal end surface facing a distal end surface of the outer peripheral part is formed on an inner surface of the outer needle hub, and at least a part of the central part is located inside the peripheral wall part.

In this way, it is possible to provide the indwelling needle in which stagnation hardly occurs in the vicinity of the elastic member by suppressing formation of a space where stagnation occurs on the distal end side of the elastic member.

The present invention includes
a holder that is attached to a proximal end of the elastic member and includes an attachment-target part to which the elastic member is attached, wherein
the elastic member includes, on a proximal end side of the solid part, a cylindrical part whose inner diameter side is supported by the attachment-target part, the cylindrical part having a radial thickness larger than an interval between the attachment-target part and a surface of the outer needle hub radially facing the attachment-target part in a state before being mounted to the outer needle hub.

In this way, a gap between the elastic member and the surface of the outer needle hub on which the elastic member is disposed is sealed by the cylindrical part compressed between the attachment-target part of the holder and the surface of the outer needle hub, so that it is possible to prevent the fluid from flowing to the proximal end side of the main body through between the elastic member and the surface of the outer needle hub on which the elastic member is disposed.

The radial thickness of the cylindrical part is not limited to a case where the thickness is constant in the axis direction, and at least a part of the cylindrical part in the axis direction may have a radial thickness larger than the interval between the attachment-target part and the surface of the outer needle hub radially facing the attachment-target part.

### Advantageous Effects of Invention

According to the present invention, it is possible to provide the indwelling needle in which stagnation hardly occurs in the vicinity of the elastic member.

### Brief Description of Drawings

FIG. 1 is a side view of an indwelling needle of a first embodiment.
FIG. 2 is a cross-sectional view taken along a direction A-A in FIG. 1.
FIG. 3 is a cross-sectional view taken along a direction B-B in FIG. 2.
FIG. 4 is an enlarged view of a main part of FIG. 3.
FIG. 5 is a cross-sectional view taken along a direction D-D in FIG. 1.
FIG. 6 is an exploded perspective view including an outer needle hub, a hemostasis valve, and a hemostasis valve holder of the first embodiment.
FIGS. 7(A) to 7(D) are drawings illustrating a configuration of an outer needle hub to which a hemostasis valve holder is mounted according to a third modification.
FIG. 8 is a side view of the hemostasis valve holder of the third modification.
FIGS. 9(A) to 9(D) are drawings illustrating a configuration of an outer needle hub to which a hemostasis valve holder is mounted according to a fourth modification.
FIGS. 10(A) and 10(B) are a side view and an end view of the hemostasis valve holder of the fourth modification.
FIG. 11 is an exploded perspective view of an outer needle hub, a hemostasis valve, and a hemostasis valve holder of a second embodiment.
FIGS. 12(A) to 12(E) are drawings illustrating a configuration of the outer needle hub to which the hemostasis valve and the hemostasis valve holder are mounted according to the second embodiment.
FIGS. 13(A) to 13(D) are drawings illustrating a configuration of an outer needle hub to which a hemostasis valve and a hemostasis valve holder are mounted according to a fifth modification.
FIG. 14 is a drawing illustrating a configuration of an outer needle hub to which a hemostasis valve holder is mounted according to a sixth modification.
FIG. 15 is a side view and an end view of the hemostasis valve holder of the sixth modification.
FIG. 16 is a plan view of an outer needle hub of a seventh modification.
FIG. 17 is a cross-sectional view taken along a direction C-C in FIG. 16.
FIG. 18 is a cross-sectional view taken along a direction E-E in FIG. 17 according to the seventh modification.
FIG. 19 is an exploded perspective view of another modification.
FIGS. 20(A) and 20(B) are a plan view and an A-A cross-sectional view of an outer needle hub to which a hemostasis valve holder is mounted according to a third embodiment.
FIGS. 21(A) and 21(B) are a side view and a B-B cross-sectional view of the outer needle hub to which the hemostasis valve holder is mounted according to the third embodiment.
FIGS. 22(A) and 22(B) are a perspective view and an A-A cross-sectional view of a hemostasis valve of the third embodiment.
FIGS. 23(A) and 23(B) are side views of the hemostasis valve holder of the third embodiment and that of a reference example.
FIGS. 24(A) and 24(B) are an A-A cross-sectional view and a B-B cross-sectional view of an outer needle hub to which the hemostasis valve holder is mounted according to the reference example.

### Description of Embodiments

Hereinafter, modes for carrying out the present invention will be exemplarily described in detail based on embodiments with reference to the drawings. However, the dimensions, materials, shapes, and relative arrangements of components described in the embodiments should be changed as appropriate according to the configuration of a device to which the invention is applied and various conditions. That is, the scope of the present invention is not intended to be limited to the following embodiments.

### (First embodiment)

### <Outline of indwelling needle>

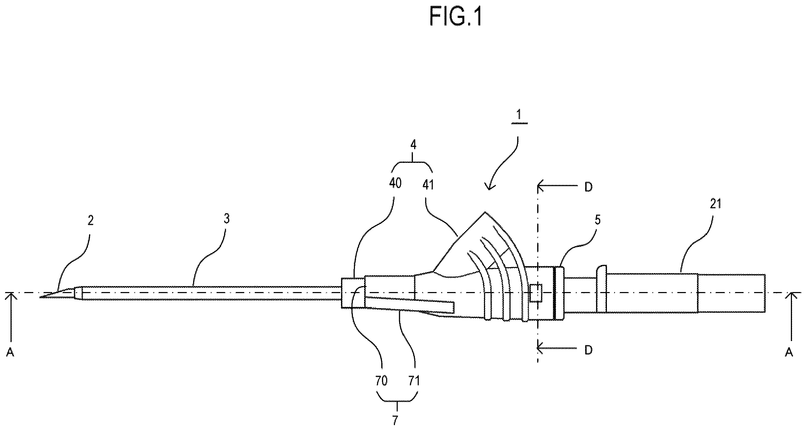

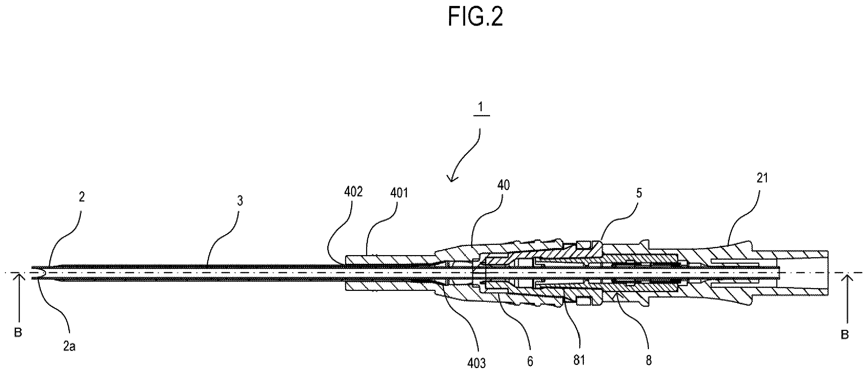

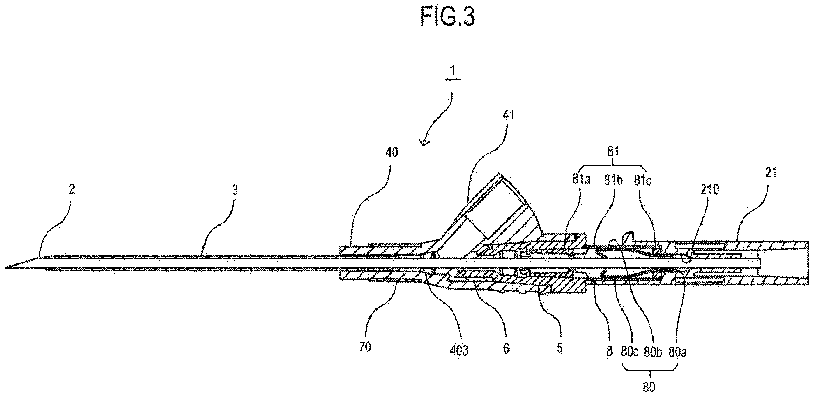

FIG. 1 illustrates a side view of an indwelling needle 1 according to an embodiment of the present invention. FIG. 2 is a cross-sectional view taken along a direction A-A of the indwelling needle 1 in FIG. 1. FIG. 3 is a cross-sectional view taken along a direction B-B in FIG. 2 of the indwelling needle 1 illustrated in FIG. 1.

First, the outline of the indwelling needle 1 will be described with reference to FIG. 1. As illustrated in FIG. 1, the indwelling needle 1 includes a hollow inner needle 2 and a cylindrical outer needle 3 provided to cover an outer surface of the inner needle 2. The inner needle 2 protrudes from an opening at a distal end of the outer needle 3, and punctures a blood vessel of a patient (living body) as a puncture object. In the following description, an end (side/direction) of the indwelling needle 1 from which the inner needle 2 protrudes is referred to as a distal end (side/direction), and an opposite end is referred to as a rear end (side/direction) or a proximal end (side/direction). A direction in which the inner needle 2 protrudes is also referred to as a puncture direction.

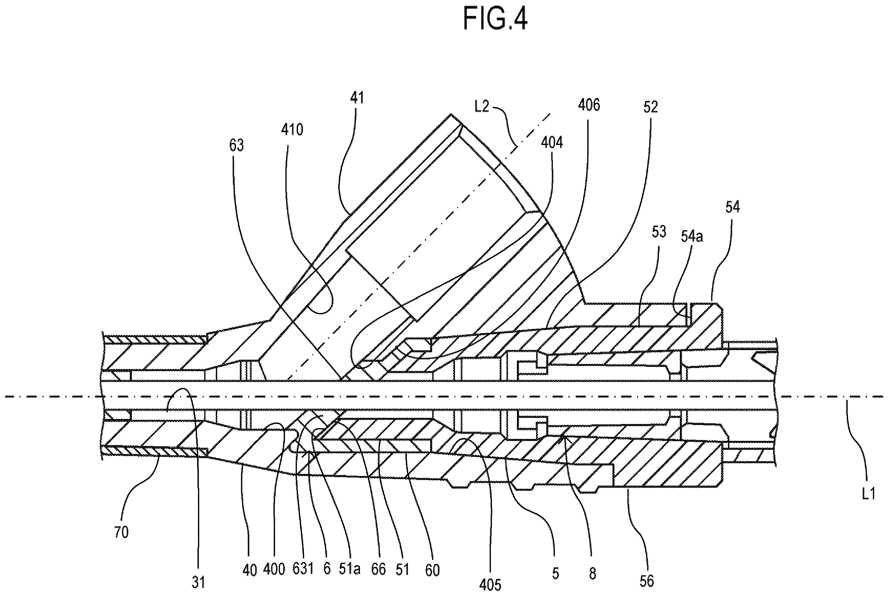

An outer needle hub 4 is also connected to a rear end of the outer needle 3. A hollow portion communicating with a hollow portion 31 of the outer needle 3 is provided inside the outer needle hub 4 (see FIG. 4). As illustrated in FIG. 1, the outer needle hub 4 includes a main body 40 extending linearly in the puncture direction, that is, a direction of an axis L1, which will be described later, of the inner needle 2, and a substantially cylindrical branch part 41 branching in a Y-shape in a direction opposite to the puncture direction from the main body 40, that is, in a direction inclined with respect to the direction of the axis L 1 of the inner needle 2 (FIG. 4 illustrates an axis L2 of the branch part 41.). A hollow portion 400 and a hollow portion 405 are provided inside the main body 40 (see FIG. 4). A hollow portion 410 is also provided inside the branch part 41 (see FIG. 4). The hollow portion 31 of the outer needle, the hollow portions 400 and 405 of the main body 40, and the hollow portion 410 of the branch part 41 communicate with each other. When the inner needle 2 is removed from the outer needle 3, a passage is formed that brings the hollow portion 31 of the outer needle 3, the hollow portion 400 of the main body 40, and the hollow portion 410 of the branch part 41 into communication. A second end surface 63 of a hemostasis valve 6, which will be described later, forms a part of the passage. The outer needle hub 4 can be formed of, for example, a resin such as polypropylene, and is not particularly limited as long as a material typically used for medical instruments is used. Note that the outer needle hub 4 may be formed of a single member or a plurality of members. In addition, a flexible soft tube may be disposed between a distal end and a proximal end of the outer needle hub 4. Furthermore, the outer needle hub 4 is not limited to the Y-shape, and may be, for example, an L-shape. Moreover, the branch part 41 may extend in a left-right direction instead of extending to the top side.

An extension tube through which, for example, blood supplied from an external apparatus such as an artificial dialysis apparatus passes is connected to a rear end of the branch part 41 in the indwelling needle 1. Note that a connector may be provided at a port end without providing the extension tube. Here, the blood corresponds to a fluid of the present invention.

A hemostasis valve holder 5 that supports the hemostasis valve 6 described later, and an inner needle hub 21 that holds a rear end of the inner needle 2 are connected to a rear end of the main body 40 in the indwelling needle 1. Here, the hemostasis valve 6 and the hemostasis valve holder 5 correspond to an elastic member and a support member of the present invention, respectively.

A pair of wings 7 is provided on the outer periphery near a distal end of the main body 40 in the indwelling needle so as to sandwich the main body 40. The wings 7 include a cylindrical sleeve 70 fitted and attached to the outer periphery near the distal end of the main body 40, and plate-like parts 71 protruding from side surfaces of the sleeve 70 in a direction perpendicular to the axis direction (the front side and the back side of the drawing). The sleeve 70 is fixed so as not to move axially nor around the axis with respect to the main body. The wings 7 function as a gripping part for an operator to grip when he/she uses the indwelling needle 1, and also function as a fixing part for fixing the indwelling needle 1 to a body with a tape or the like. Although the wings extend in a direction intersecting the extending direction of the branch part, the wings may extend in a direction parallel to the branch part. In addition, the sleeve 70 may be provided so as to be relatively rotatable in a direction around the axis with respect to the main body. In this case, a user can place the wings at a suitable position. Furthermore, the wings do not have to be provided.

### <Internal configuration of indwelling needle>

Next, the details of the inside of the indwelling needle 1 will be described using FIGS. 2 and 3. As illustrated in FIG. 2, an outer needle holding part 401 that holds the proximal end side of the outer needle 3 is provided on the distal end side of the main body 40 of the outer needle hub 4. A distal end opening 402 that opens in the puncture direction is provided at a distal end of the outer needle holding part 401. The outer needle 3 is held by the outer needle holding part 401 such that its distal end protrudes from the distal end opening 402. The proximal end side of the outer needle 3 is fixed by a caulking pin 403 filling a space between the outer needle 3 and the outer needle hub 4.

In addition, the hemostasis valve 6 is attached to a distal end of the hemostasis valve holder 5. The inner needle hub 21 is connected to a rear end side of the hemostasis valve holder 5. The inner needle hub 21 holds the proximal end side of the inner needle 2 on its distal end side. A protector 8 for protecting a needle tip 2a of the inner needle 2 is attached to the distal end side of the inner needle hub 21. The protector 8 includes a protector body 80 and a protector holder 81.

The protector body 80 is configured to cover and protect the needle tip 2a of the inner needle 2, and is made of various metal materials such as stainless steel, aluminum, an aluminum alloy, a titanium alloy, copper, a copper alloy, and a copper-based alloy, for example. The protector body 80 can be configured as, for example, a substantially quadrangular prism-shaped member obtained by bending and deforming a thin plate made of the above-described metal material. As illustrated in FIG. 3, the protector body 80 mainly includes a substantially square bottom plate part 80a and flexible protective pieces 80b and 80c extending from two opposing sides of the bottom plate part 80a toward a distal end. The inner needle 2 is inserted into a hole opened at the center of the bottom plate part 80a, and the protector body 80 is supported so as to be movable in the axis direction of the inner needle 2. The protective piece 80b and the protective piece 80c are substantially rectangular plate-like members, and face each other with the inner needle 2 interposed therebetween. The protective piece 80b and the protective piece 80c have a substantially J-shape in which the pieces are bent in a direction away from each other from the bottom plate part 80a toward the distal end side (an outer diameter direction of the inner needle 2), then bent in a direction approaching each other (an inner diameter direction of the inner needle 2), and bent in a direction approaching each other toward the rear end side on the distal end side. The protective piece 80b is formed to be longer than the protective piece 80c, and a distal end part of the protective piece 80b is located closer to the distal end than a distal end part of the protective piece 80c.

The protector holder 81 is a substantially cylindrical member made of a synthetic resin. The protector holder 81 has a cylindrical part 81a on the distal end side, a housing part 81b on the rear end side, and a ring part 81c at a rear end part of the housing part 81b. The cylindrical part 81a is formed in a substantially cylindrical shape. The cylindrical part 81a is inserted from an opening on the rear end side of the hemostasis valve holder 5, is housed in a hollow portion of the hemostasis valve holder 5, and is locked to the hemostasis valve holder 5 by engagement means such as unevenness (not illustrated). The housing part 81b is formed in a quadrangular tube shape having a substantially square cross section, and its distal end side is formed integrally with a rear end of the cylindrical part 81a. Substantially rectangular openings are formed in a pair of opposing side walls (two side walls in a top-bottom direction in FIG. 3) of the housing part 81b. These openings are provided on the side opposite to the inner needle 2 with respect to the protective piece 80b and the protective piece 80c. These openings allow deformation of the protective piece 80b and the protective piece 80c in the direction away from each other.

In addition, the ring part 81c is formed in an annular shape having a square cross section composed of four side walls. The dimensions of an inner peripheral part of the ring part 81c are formed to substantially coincide with the outer dimensions of the bottom plate part 80a of the protector body 80. As a result, when the protector body 80 is moved so as to be pulled out from the ring part 81c, inserting the protective piece 80b and the protective piece 80c of the protector body 80 into the ring part 81c, the protective piece 80b and the protective piece 80c are pushed inward by the ring part 81c. By being pushed inward, the protective piece 80b and the protective piece 80c are elastically deformed so as to approach each other, and the protective piece 80b and the protective piece 80c facing each other come into abutment with each other, thereby closing a gap therebetween. At this time, deformed portions of the protective piece 80b and the protective piece 80c protrude from the openings of the housing part 81b. The protective piece 80b and the protective piece 80c cannot move in the direction of approaching each other any more as described above. Therefore, the movement of the protector body 80 is restricted by rear end side portions of the protective piece 80b and the protective piece 80c, and the protector body 80 cannot be removed from the protector holder 81. In this way, by moving the protector body 80 in the direction of pulling out the protector body 80 from the protector holder 81, the needle tip 2a of the inner needle 2 is covered and protected by the protector body 80, and cannot move relative to the protector holder 81, whereby the protected state is maintained.

When the outer needle 3 is indwelled in the body and the inner needle hub 21 is pulled out from the outer needle hub 4, an engagement part provided on the distal end side of the inner needle 2 and the bottom plate part 80a of the protector body 80 are engaged with each other since at least a part of the outer diameter of the engagement part is set to be larger than the inner diameter of the hole opened in the bottom plate part 80a. When the inner needle hub 21 is further pulled out, the protector body 80 including the bottom plate part 80a engaged with the inner needle 2 moves to the rear end side. When the inner needle hub 21 is further pulled out, the protector body 80 moves to the rear end side relative to the protector holder 81 as described above. Therefore, the protective piece 80b and the protective piece 80c abutting on each other can cover and protect the needle tip 2a of the inner needle 2.

### <Configuration of main part>

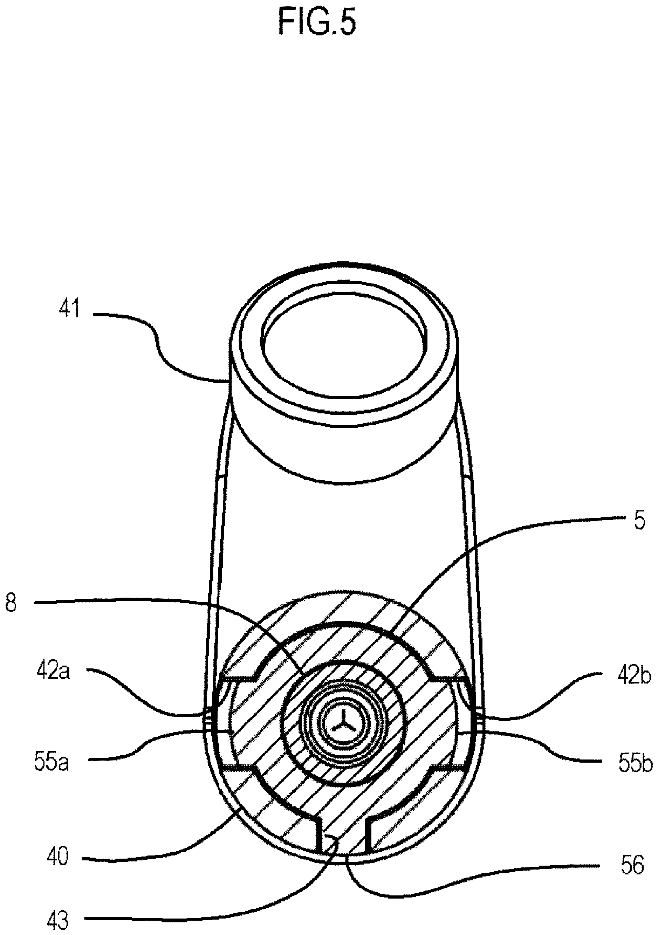

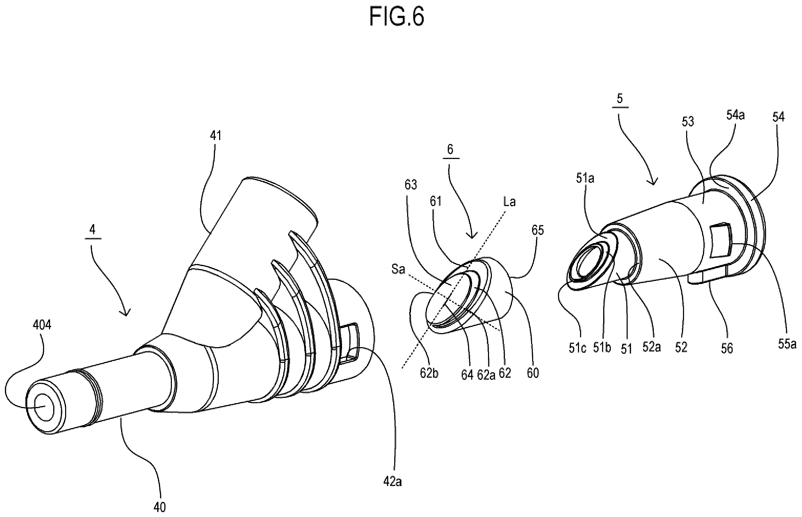

FIG. 4 is an enlarged view illustrating a main part of the cross-sectional view illustrated in FIG. 3. FIG. 5 is a cross-sectional view taken along a direction D-D in FIG. 1. FIG. 6 is an exploded perspective view of the outer needle hub 4, the hemostasis valve 6, and the hemostasis valve holder 5. The axis L1 is a line indicating the axis direction of the main body 40 of the outer needle hub 4, and coincides with the axis of the inner needle 2 in a state where the inner needle 2 is inserted into the outer needle 3.

As illustrated in FIG. 4, the outer needle hub 4 is provided with the hollow portion 400 communicating with the hollow portion 31 of the outer needle 3. The hollow portion 405 on the rear end side in the axis L1 direction from the hollow portion 400 and the hollow portion 410 inclined with respect to the axis L1 direction from the hollow portion 400 communicate with each other via the hollow portion 400.

The hemostasis valve 6 and the hemostasis valve holder 5 are attached to the hollow portion 405. The inner needle hub 21 equipped with the protector 8 is attached to the rear end side of the hemostasis valve holder 5. At this time, the inner needle 2 pushes open a slit 64 of the hemostasis valve 6 to be inserted into the hollow portion 31 of the outer needle 3. The hemostasis valve 6 is formed of, for example, synthetic rubber such as polyisoprene, or thermoplastic elastomer. The hemostasis valve holder 5 can be formed of, for example, a resin such as polypropylene, and is not particularly limited as long as a material typically used for medical instruments is used.

As illustrated in FIG. 6, the hemostasis valve 6 is formed in a substantially bottomed cylindrical shape. An end part on the distal end side of the hemostasis valve 6 is formed in an inclined surface inclined with respect to the axis L1 direction. Specifically, the hemostasis valve 6 includes a cylindrical part 60, an annular first end surface 61 connected to an end edge on the distal end side of the cylindrical part 60, an annular step part 62 connected to the inner diameter side of the first end surface 61 and extending in the axis L1 direction, and a disk-shaped second end surface 63 connected to the inner diameter side of the step part 62. The first end surface 61 and the second end surface 63 of the hemostasis valve 6 are configured to be substantially parallel, and are similarly inclined with respect to the axis L1 direction. The linear slit 64 penetrating a soft solid part 631 from the distal end side to the rear end side is opened at the center of the second end surface 63. Note that the slit may also have a cross shape, a Y-shape, or the like. In addition, a central proximal end surface 66, which is a surface on the rear end side of the first end surface 61 and the second end surface 63 of the hemostasis valve 6, is formed as a single surface parallel to the first end surface 61. The shape of the second end surface 63 of the hemostasis valve 6 is not limited to a flat surface, and may be formed into a curved surface conforming to the shape of a cylindrical surface of a peripheral wall of the hollow portion 410 of the branch part 41. It can also be said that the second end surface 63 is a central part of the hemostasis valve 6, and the first end surface 61 is an outer peripheral part formed on the outer periphery of the central part. In addition, in the hemostasis valve 6, the second end surface 63 and the step part 62 may be formed in a shape in which a straight line La indicated by a dotted line in FIG. 4 is a long axis and a straight line Sa is a short axis. At this time, an opening 404 of the outer needle hub 4 is similarly formed in a shape having a long axis and a short axis. The central part of the hemostasis valve 6 and the opening 404 of the outer needle hub 4 have the long axes and the short axes, to function as an outer peripheral part and a guided part, so that the hemostasis valve 6 can be easily positioned in a predetermined direction around the axis. In addition, in a case where the second end surface 63 and the step part 62 are formed in the shape having the long axis and the short axis as described above, it is desirable to set the long axis in a direction along the axis L2 direction of the branch part 41 from the viewpoint of miniaturization. Furthermore, when the second end surface 63 has the shape having the long axis and the short axis as described above, it is desirable to form the linear slit along the long axis direction. The step part 62 formed between the first end surface 61 and the second end surface 63 is inclined with respect to the axis L 1 direction, and is easily inserted when the central part of the hemostasis valve 6 is inserted into the opening 404 of the outer needle hub 4. In addition, the hemostasis valve 6 is opened in a cylindrical shape on the rear end side, and the center is thinned relative to the outer peripheral part to reduce resistance at the time of pulling out the inner needle. An end surface 65 on the rear end side of the hemostasis valve 6 forms a plane perpendicular to the axis L1 direction, and abuts on a step part 52a of the hemostasis valve holder 5. In this way, it is possible to reduce unintentional deformation of an elastic valve body when the hemostasis valve 6 is relatively moved in the outer needle hub.

The hemostasis valve holder 5 is formed in a substantially cylindrical shape. The hemostasis valve holder 5 includes a small diameter part 51, a diameter expanded part 52, a large diameter part 53, and a flange part 54 sequentially from the distal end side. An annular first end surface 51a that is connected to the inner diameter side of an end edge of the small diameter part 51 and is inclined with respect to the axis L1 direction is formed at an end part on the distal end side of the small diameter part 51. An annular step part 51b is formed that is connected to the inner diameter side of the first end surface 51a and extends in the axis L1 direction. A second end surface 51c is also formed that is connected to the inner diameter side of the step part and is inclined with respect to the axis L1 direction. The first end surface 51a and the second end surface 51c of the hemostasis valve holder 5 are configured to be substantially parallel, and are similarly inclined with respect to the axis L1 direction. In addition, the first end surface 51a and the second end surface 51c of the hemostasis valve holder 5 are configured to be parallel to the first end surface 61 and the second end surface 63 of the hemostasis valve 6, respectively, and are similarly inclined with respect to the axis L1 direction. Here, the annular step part 51b may be provided so as to be inclined with respect to the axis L1 direction in order to increase a contact area with the hemostasis valve 6. In addition, a distal end part of the second end surface 51c is desirably formed in a rounded shape.

In the hemostasis valve holder 5, the rear end side of the small diameter part 51 is connected to the diameter expanded part 52 having a diameter increasing toward the rear end side via the step part 52a extending to the outer diameter side, that is, in a direction perpendicular to the axis L1. The rear end side of the diameter expanded part 52 is connected to the large diameter part 53. The annular flange part 54 protruding to the outer diameter side is formed on the rear end side of the large diameter part 53. Formed on side surfaces of the large diameter part 53 are a protrusion 55a and a protrusion 55b (see FIG. 5) that protrude to the outer diameter side gradually from the distal end side toward the rear end side. The protrusion 55a and the protrusion 55b are provided at symmetrical positions with respect to the axis L1. A rib 56 protruding to the outer diameter side and extending in the axis L1 direction from the distal end side toward the rear end side is also formed on the side surface of the large diameter part 53, and the rear end side of the rib 56 is connected to the flange part 54. The rib 56 is provided between the protrusion 55a and the protrusion 55b, and is provided at a position opposite to the branch part 41 with respect to the axis L1 direction in relation with the outer needle hub 4.

The outer diameter dimension of the small diameter part 51 of the hemostasis valve holder 5 is set to be slightly smaller than the inner diameter dimension of the cylindrical part 60 of the hemostasis valve 6. When the small diameter part 51 of the hemostasis valve holder 5 is fitted into the inner periphery of the cylindrical part 60 of the hemostasis valve 6 from the distal end side, the cylindrical part 60 of the hemostasis valve 6 slightly increases in diameter to the outer diameter side, but tries to decrease in diameter to be elastically deformed. The elastic deformation produces a frictional force between an inner peripheral surface of the cylindrical part 60 of the hemostasis valve 6 and an outer peripheral surface of the small diameter part 51 of the hemostasis valve holder 5. Since the first end surface 51a, which is a distal end surface of the hemostasis valve holder 5, is inclined with respect to the axis direction similarly to the central proximal end surface 66 of the hemostasis valve 6, the first end surface 51a restricts displacement of the hemostasis valve 6 with respect to the hemostasis valve holder 5 in the axis L1 direction and a rotation direction about the axis L1, and the first end surface 51a also functions as a guide in mounting the hemostasis valve 6 and the hemostasis valve holder 5 in a predetermined direction around the axis. The dimensions in the axis L1 direction of the cylindrical part 60 of the hemostasis valve 6 and the small diameter part 51 of the hemostasis valve holder 5 are set such that the end surface 65 on the rear end side of the cylindrical part 60 of the hemostasis valve 6 abuts on the step part 52a of the hemostasis valve holder 5 in a state where the first end surface 51a of the small diameter part 51 of the hemostasis valve holder 5 abuts on the rear end side of the first end surface 61 and the second end surface 63 of the hemostasis valve 6. When the small diameter part 51 of the hemostasis valve holder 5 is pushed into the hemostasis valve 6 to a position where the step part 52a of the hemostasis valve holder 5 abuts on the end surface on the rear end side of the cylindrical part 60 of the hemostasis valve 6, the step part 51b and the second end surface 51c of the small diameter part 51 of the hemostasis valve holder 5 slightly bite into the rear end side of the second end surface 63 of the hemostasis valve 6. A hollow portion of the hemostasis valve 6 and the small diameter part 51 of the hemostasis valve holder 5 are configured in shapes rotationally asymmetric to each other with respect to the axis L1. Thus, the rotation of the hemostasis valve 6 about the axis L1 with respect to the hemostasis valve holder 5 is restricted in a state where the hemostasis valve 6 is attached to a predetermined position of the small diameter part 51 of the hemostasis valve holder 5. Such a rotation preventing mechanism that restricts relative rotation between the hemostasis valve holder 5 and the hemostasis valve 6 is provided, so that it is possible to reduce the occurrence of displacement of the hemostasis valve at the time of mounting. Note that the shape of the small diameter part of the hemostasis valve holder 5 is not limited to the cylindrical shape.

FIG. 4 illustrates a state in which the hemostasis valve 6 and the hemostasis valve holder 5 are appropriately positioned with respect to the outer needle hub 4. The opening 404 is provided on the distal end side of the hollow portion 405 of the main body 40 of the outer needle hub 4 via a step part 406. The inner diameter of the opening 404 in the direction perpendicular to the axis L1 is set to be smaller than the inner diameter of the hollow portion 405. The inner diameter of the hollow portion 405 is set such that an outer peripheral surface of the hemostasis valve holder 5 to which the hemostasis valve 6 is attached is in pressure contact with an inner peripheral surface of the hollow portion 405 to block the flow of a liquid such as blood through the outer peripheral sides of the hemostasis valve 6 and the hemostasis valve holder 5 between the hollow portion 400 and the hollow portion 405. To this end, the inner peripheral surface on the distal end side of the hollow portion 405 is formed in a cylindrical shape corresponding to the cylindrical part 60 of the hemostasis valve 6, the rear end side thereof is formed in a tapered shape having a diameter increasing to the outer diameter side toward the rear end side corresponding to the diameter expanded part 52 of the hemostasis valve holder 5, and the further rear end side thereof is formed in a cylindrical shape corresponding to the large diameter part 53 of the hemostasis valve holder 5. Note that a portion including the step part 406 and the opening 404 of the outer needle hub 4 may be formed as a separate member from the other portions of the outer needle hub 4, and may be integrated by fixing the separate member to the outer needle hub 4.

When the hemostasis valve 6 and the hemostasis valve holder 5 are appropriately positioned with respect to the outer needle hub 4 as illustrated in FIG. 4, the first end surface 61 and the step part 62 of the hemostasis valve 6 abut on inner peripheral surfaces of the step part 406 and the opening 404 of the outer needle hub 4, respectively, and the hemostasis valve 6 is compressively fixed in the axis L1 direction between the step part 406 of the outer needle hub 4 and the first end surface 51a of the hemostasis valve holder 5 facing each other. The second end surface 63 of the hemostasis valve 6 is exposed to the hollow portion 400. The second end surface 63 of the hemostasis valve 6 is set to substantially the same height as a portion adjacent to the opening 404 in an inner peripheral surface of the hollow portion 410 of the branch part 41. As described above, in the hemostasis valve 6, the outer peripheral part is firmly fixed, and the central part fills an air gap in the outer needle hub 4. In the hollow portion 410 branching from the hollow portion 400, there is no rapid shape change in a part of the second end surface 63 of the hemostasis valve 6 constituting a part of the inner peripheral surface of the hollow portion 410. Therefore, a liquid such as blood smoothly flows in the hollow portion 31 of the outer needle 3, and the hollow portion 400 of the main body 40 and the hollow portion 410 of the branch part 41 of the outer needle hub 4, generating no stagnation that causes blood clots. The step part 406 and the opening 404 of the outer needle hub 4 function as a peripheral wall part for the second end surface 63 as the central part on the distal end side of the hemostasis valve 6, and the step part 406 constitutes a proximal end surface of the peripheral wall part engaged with the first end surface. The step part 406 inclined with respect to the axis L1 direction constitutes a guiding part, and the distal end surface of the hemostasis valve 6 constitutes a guided part.

In addition, when the hemostasis valve 6 is appropriately positioned with respect to the outer needle hub 4, an interval between parts 62a and 62b facing each other across the slit 64, of the step part 62 of the hemostasis valve 6 is set to be slightly larger than the inner diameter of a corresponding part of the opening 404, and a portion of the hemostasis valve 6 including these parts 62a and 62b is interference-fitted to the opening 404. With this setting, a compressive force in a direction in which the slit 64 of the hemostasis valve 6 is closed is applied by the opening 404 of the outer needle hub 4, so that an elastic return force of the slit 64 at the time of removing the inner needle 2 and after removing the inner needle 2 is increased, and the sealing property of the hemostasis valve 6 can be ensured.

In the above embodiment, the hemostasis valve 6 is compressed to the inner diameter side by the inner periphery of the opening 404 as described above. However, the compression mode is not limited to the above mode. For example, it is also possible to ensure the sealing property of the hemostasis valve 6 by forming the hemostasis valve 6 to be thick such that the central proximal end surface 66 of the hemostasis valve 6 is perpendicular to the axis direction, and compressing the hemostasis valve 6 radially inward from the outer needle hub 4. In addition, the thickness of the hemostasis valve 6 can be further increased to ensure the sealing property of the hemostasis valve 6 without compressing the hemostasis valve 6. Furthermore, an inclined surface inclined with respect to the axis direction may be provided at a contact part of the hemostasis valve holder 5 with the outer needle hub 4, and an inclined surface engaged with the inclined surface may be provided on an inner surface of the outer needle hub 4, to constitute the guiding part and the guided part, and the direction around the axis of the outer needle hub 4 and the hemostasis valve holder 5 may be set as a prescribed direction.

An opening 42a and an opening 42b having a rectangular shape are opened on side surfaces on the rear end side of the main body 40 of the outer needle hub 4. The opening 42a and the opening 42b are provided at symmetrical positions with respect to the axis L1. In addition, a cutout 43 whose rear end side is released is opened on the rear end side of the main body 40 of the outer needle hub 4. The cutout 43 is provided between the opening 42a and the opening 42b, and is provided at a position opposite to the branch part 41 with respect to the axis L1.

When the hemostasis valve holder 5 to which the hemostasis valve 6 is attached is press-fitted from an opening on the rear end side of the outer needle hub 4 to be attached to the outer needle hub 4, the protrusion 55a, the protrusion 55b, and the rib 56 of the hemostasis valve holder 5 are held in a posture corresponding to the opening 42a, the opening 42b, and the cutout 43 of the outer needle hub 4. The hemostasis valve holder 5 is advanced in the axis L1 direction with respect to the outer needle hub 4 to be press-fitted from the opening on the rear end side, and the protrusion 55a, the protrusion 55b, and the rib 56 are fitted to the opening 42a, the opening 42b, and the cutout 43, respectively. At this time, since the cutout 43 and the rib 56 extend in the axis L1 direction, the hemostasis valve holder 5 is guided in the axis L1 direction with respect to the outer needle hub 4 by the cutout 43 and the rib 56.

In this way, the direction around the axis L1 of the hemostasis valve 6 and the hemostasis valve holder 5 with respect to the outer needle hub 4 when the hemostasis valve 6 and the hemostasis valve holder 5 are attached to the outer needle hub 4 can be set to a predetermined direction. That is, the direction around the axis L 1 of the hemostasis valve 6 and the hemostasis valve holder can be guided in a certain direction, whereby the direction around the axis L1 of the hemostasis valve 6 attached to the hemostasis valve holder 5 can be defined. By providing the rotation preventing mechanism including the protrusion 55a, the protrusion 55b, the rib 56, the opening 42a, the opening 42b, and the cutout 43 as described above, it is possible to prevent the hemostasis valve 6 and the hemostasis valve holder 5 from rotating about the axis L1 with respect to the outer needle hub 4. Note that the rotation preventing mechanism may be composed of only the rib 56 and the cutout 43. In addition, the protrusion 55a and the protrusion 55b of the hemostasis valve holder 5 are fitted to the opening 42a and the opening 42b of the outer needle hub 4 to constitute a locking part that restricts relative movement in the axis L1 direction with respect to the outer needle hub 4.

### <First modification>

In the above-described embodiment, the example has been described in which the rotation preventing mechanism in attaching the hemostasis valve 6 and the hemostasis valve holder 5 to the outer needle hub 4 is provided in the hemostasis valve holder 5 and the outer needle hub 4. Here, the protrusion 55a, the protrusion 55b, and the rib 56 are provided in the hemostasis valve holder 5, and the opening 42a, the opening 42b, and the cutout 43 are provided in the outer needle hub 4. On the other hand, an opening, a cutout, or a recess may be provided in the hemostasis valve holder 5, and a protrusion or a rib may be provided on the inner peripheral surface of the hollow portion 405 of the outer needle hub 4.

### <Second modification>

In the modification of the above-described embodiment, the central proximal end surface 66 of the hemostasis valve 6 and the first end surface 51a at the distal end of the hemostasis valve holder 5 are inclined, restricting the relative rotation between the hemostasis valve 6 and the hemostasis valve holder 5. However, the central proximal end surface 66 of the hemostasis valve 6 and the first end surface 51a at the distal end of the hemostasis valve holder 5 do not have to be inclined. The relative rotation may be restricted by providing a cutout, a recess, a protrusion, or the like in the hemostasis valve and providing a protrusion or the like engaged with the cutout, the recess, or the protrusion in the hemostasis valve holder. In addition, by roughening one or both of the inner peripheral surface of the cylindrical part 60 of the hemostasis valve 6 and the outer peripheral surface of the small diameter part 51 of the hemostasis valve holder 5, a frictional force may be applied between the hemostasis valve 6 and the small diameter part 51 of the hemostasis valve holder 5. Furthermore, the hemostasis valve 6 and the small diameter part 51 of the hemostasis valve holder 5 may be formed of materials that generate a frictional force with each other. A member that generates a frictional force may be interposed between the hemostasis valve 6 and the small diameter part 51 of the hemostasis valve holder 5. The hemostasis valve 6 and the small diameter part 51 of the hemostasis valve holder 5 may be fixed with an adhesive. The cause of the frictional force between the hemostasis valve 6 and the small diameter part 51 of the hemostasis valve holder 5 is not limited to an intermolecular force.

### <Third modification>

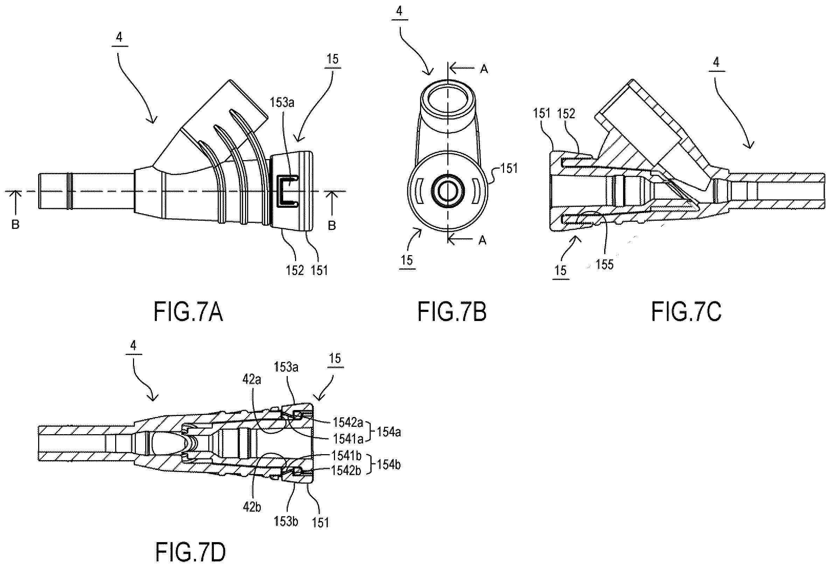

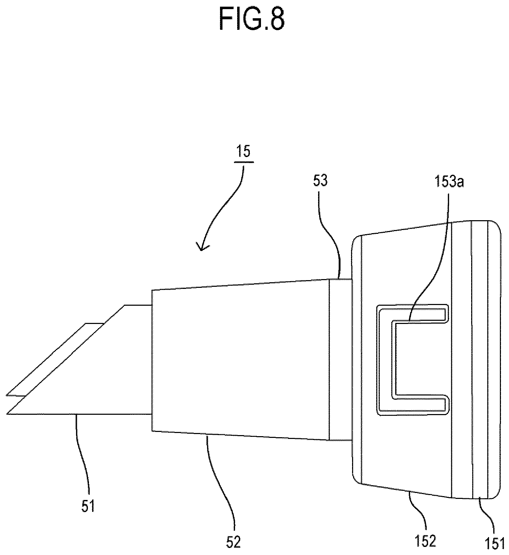

With reference to FIGS. 7 and 8, the outer needle hub 4 and a hemostasis valve holder 15 according to a third modification, which is a modification of the first embodiment, will be described. Since the hemostasis valve 6 and other components of the indwelling needle are similar to those of the above-described embodiment, the detailed description thereof will be omitted. The same reference numerals are used for the same components as those of the first embodiment.

FIG. 7(A) is a side view of the outer needle hub 4 to which the hemostasis valve holder 15 is mounted, and FIG. 7(B) is an end view of the outer needle hub 4 to which the hemostasis valve holder 15 is mounted as viewed from the proximal end side along the axis L1 direction. FIG. 7(C) is a cross-sectional view taken along a direction A-A of the outer needle hub 4 to which the hemostasis valve holder 15 is mounted, and FIG. 7(D) is a cross-sectional view taken along a direction B-B of the outer needle hub 4 to which the hemostasis valve holder 15 is mounted. FIG. 8 is a side view of the hemostasis valve holder 15.

As illustrated in FIG. 8, the hemostasis valve holder 15 includes the small diameter part 51, the diameter expanded part 52, and the large diameter part 53 similarly to the first embodiment. In the third modification, as illustrated in FIGS. 7(C) and 7(D), a flange-shaped end part 151 protruding to the outer diameter side, and an outer insertion part 152 having a substantially cylindrical shape, extending from an edge on the outer diameter side of the end part 151 toward the distal end side so as to cover the outer peripheral side of the large diameter part 53 in parallel with the large diameter part 53 are provided on the rear end side of the large diameter part 53. The outer insertion part 152 is provided with a claw 153a and a claw 153b at symmetrical positions with respect to the axis L1. The substantially rectangular claws 153a and 153b are joined to the outer insertion part 152 on the rear end side, and are provided to be elastically deformable in the radial direction with the rear end side as a fulcrum. Provided on the inner diameter side of the claw 153a and the claw 153b are a protrusion 154a and a protrusion 154b having a triangular cross section, including an inclined surface 1541a and an inclined surface 1541b protruding to the inner diameter side from the distal end side toward the rear end side, and an end surface 1542a and an end surface 1542b on the rear end side perpendicular to the axis L1 direction.

As illustrated in FIGS. 7(A) and 7(D), the opening 42a and the opening 42b having a rectangular shape are provided on the rear end side of the main body 40 of the outer needle hub 4, similarly to the embodiment. A cylindrical gap 155 is formed between the outer insertion part 152 and the large diameter part 53 of the hemostasis valve holder 15. When the small diameter part 51 of the hemostasis valve holder 15 is press-fitted into the opening at the rear end of the outer needle hub 4, the main body 40 of the outer needle hub 4 is fitted to the gap 155 from the rear end side. At this time, the inclined surface 1541a and the inclined surface 1541b abut on the main body 40 of the outer needle hub 4, so that the claw 153a and the claw 153b are elastically deformed to the outer diameter side, and the protrusion 154a and the protrusion 154b rub on an outer surface of the main body 40 of the outer needle hub 4. When the protrusion 154a and the protrusion 154b reach the positions of the opening 42a and the opening 42b, the protrusion 154a and the protrusion 154b are fitted to the opening 42a and the opening 42b, respectively, by elastic return of the claw 153a and the claw 153b.

As described above, the protrusion 154a and the protrusion 154b of the hemostasis valve holder 15 are fitted to the opening 42a and the opening 42b of the outer needle hub 4 from the outer diameter side, so that the direction around the axis L1 of the hemostasis valve holder 15 can be guided in a certain direction. Therefore, the direction around the axis L1 of the hemostasis valve 6 attached to the hemostasis valve holder 15 can be defined. Consequently, blood flowing into the outer needle hub 4 from the outer needle 3 can be smoothly moved to the branch part 41, and stagnation in the vicinity of the hemostasis valve 6 can be prevented.

Here, the opening 42a and the opening 42b of the outer needle hub 4 constitute the guiding part. The protrusion 154a and the protrusion 154b constitute the guided part, and the opening 42a and the opening 42b of the outer needle hub 4 and the protrusion 154a and the protrusion 154b of the hemostasis valve holder 15 constitute the rotation preventing mechanism.

In addition, the protrusion 154a and the protrusion 154b of the hemostasis valve holder 15 are fitted to the opening 42a and the opening 42b of the outer needle hub 4 to constitute the locking part that restricts the relative movement in the axis L1 direction with respect to the outer needle hub 4. Note that the opening 42a and the opening 42b may be recesses formed on the outer surface of the outer needle hub 4.

In the third modification, the outer needle hub 4 is provided with the opening 42a and the opening 42b, and the hemostasis valve holder 15 is provided with the protrusion 154a and the protrusion 154b. However, the outer needle hub 4 may be provided with a protrusion, and the hemostasis valve holder 15 may be provided with an opening. The protrusion of the outer needle hub 4 and the opening of the hemostasis valve holder 15 are fitted together, so that the direction around the axis L1 of the hemostasis valve holder 15 can be guided in a certain direction. Therefore, the direction around the axis L1 of the hemostasis valve 6 attached to the hemostasis valve holder 15 can be defined. Consequently, blood flowing into the outer needle hub 4 from the outer needle 3 can be smoothly moved to the branch part 41, and stagnation in the vicinity of the hemostasis valve 6 can be prevented.

### <Fourth modification>

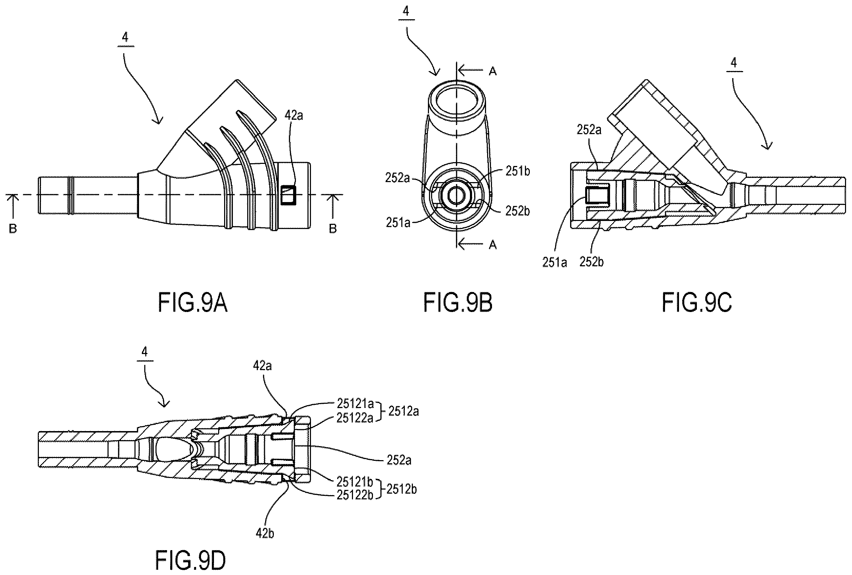

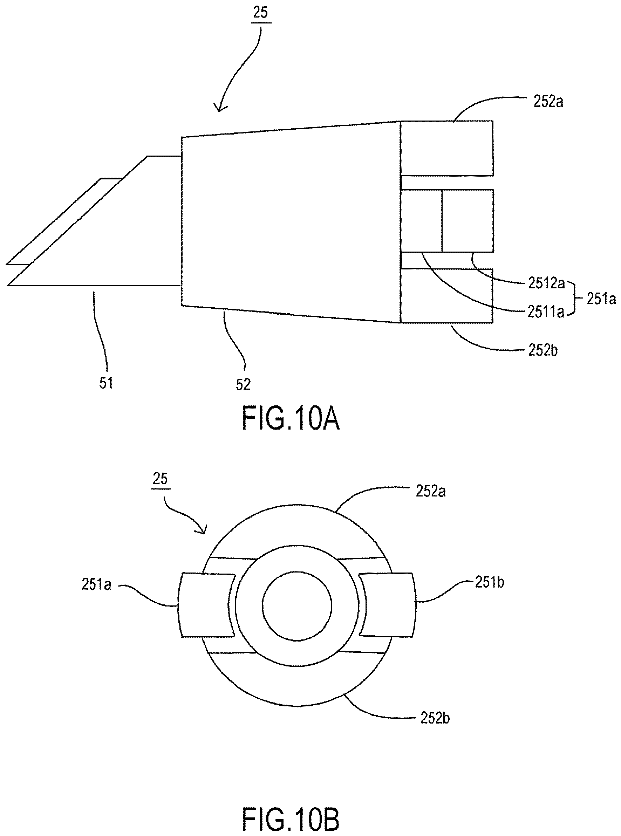

With reference to FIGS. 9 and 10, the outer needle hub 4 and a hemostasis valve holder 25 according to a fourth modification will be described. Since the hemostasis valve 6 and other components of the indwelling needle are similar to those of the above-described embodiment, the detailed description thereof will be omitted. The same reference numerals are used for the same components as those of the above-described embodiment.

FIG. 9(A) is a side view of the outer needle hub 4 to which the hemostasis valve holder 25 is mounted, and FIG. 9(B) is an end view of the outer needle hub 4 to which the hemostasis valve holder 25 is mounted as viewed from the proximal end side along the axis L1. FIG. 9(C) is a cross-sectional view taken along a direction A-A of the outer needle hub 4 to which the hemostasis valve holder 25 is mounted, and FIG. 9(D) is a cross-sectional view taken along a direction B-B of the outer needle hub 4 to which the hemostasis valve holder 25 is mounted. FIG. 10(A) is a side view of the hemostasis valve holder 25, and FIG. 10(B) is an end view of the hemostasis valve holder 25 as viewed from the proximal end side.

As illustrated in FIG. 10(A), the hemostasis valve holder 25 includes the small diameter part 51 and the diameter expanded part 52 similarly to the first embodiment. In the fourth modification, as illustrated in FIG. 10(A), a claw 251a and a claw 251b are provided at symmetrical positions with respect to the axis L1 on the rear end side of the diameter expanded part 52. The claw 251a and the claw 251b extend in the axis L1 direction from a rear end part of the diameter expanded part 52. The claw 251a and the claw 251b have a base part 2511a and a base part 2511b connected to the rear end part of the diameter expanded part 52 and having a partially cylindrical shape conforming to the shape of the rear end part, and a protrusion 2512a and a protrusion 2512b protruding to the outer diameter side on the rear end side of the base part 2511a and the base part 2511b, respectively. The protrusion 2512a forms a triangular cross-sectional shape having an inclined surface 25121a protruding to the outer diameter side from the distal end side toward the rear end side, and an end surface 25122a connected to the rear end side of the inclined surface 25121a and extending in the direction perpendicular to the axis L1 direction. Similarly, the protrusion 2512b forms a triangular cross-sectional shape having an inclined surface 25121b and an end surface 25122b. In the hemostasis valve holder 25, a partial cylindrical part 252a and a partial cylindrical part 252b are provided at symmetrical positions with respect to the axis L1. The partial cylindrical part 252a and the partial cylindrical part 252b are circumferentially sandwiched between the claws 251a and 251b and are connected to the rear end part of the diameter expanded part 52, conforming to the shape of the rear end part. A gap is formed between each of the claw 251a and the claw 251b and each of the partial cylindrical part 252a and the partial cylindrical part 252b in the circumferential direction.

As illustrated in FIGS. 9(A) and 9(D), the opening 42a and the opening 42b having a rectangular shape are provided on the rear end side of the main body 40 of the outer needle hub 4, similarly to the embodiment. When the small diameter part 51 of the hemostasis valve holder 25 is press-fitted into the opening at the rear end of the outer needle hub 4, the rear end part of the outer needle hub 4 rubs on the inclined surface 25121a of the protrusion 2512a and the inclined surface 25121b of the protrusion 2512b provided on the claw 251a and the claw 251b, and the apexes of the protrusion 2512a and the protrusion 2512b abut on the inner peripheral surface of the outer needle hub 4. Therefore, the claw 251a and the claw 251b are pressed to the inner diameter side and are elastically deformed to the inner diameter side with the base parts as a fulcrum. When the hemostasis valve holder 25 is further pushed into the outer needle hub 4 toward the distal end side and the protrusion 2512a and the protrusion 2512b reach the positions of the opening 42a and the opening 42b of the outer needle hub 4, the protrusion 2512a and the protrusion 2512b are fitted to the opening 42a and the opening 42b, respectively, from the inner diameter side by elastic return of the claw 251a and the claw 251b.

As described above, the protrusion 2512a and the protrusion 2512b of the hemostasis valve holder 25 are fitted to the opening 42a and the opening 42b of the outer needle hub 4 from the inner diameter side, so that the direction around the axis L1 of the hemostasis valve holder 25 can be guided in a certain direction. Therefore, the direction around the axis L1 of the hemostasis valve 6 attached to the hemostasis valve holder 25 can be defined. Consequently, blood flowing into the outer needle hub 4 from the outer needle 3 can be smoothly moved to the branch part 41, and stagnation in the vicinity of the hemostasis valve 6 can be prevented.

Here, the opening 42a and the opening 42b of the outer needle hub 4 constitute the guiding part. The protrusion 2512a and the protrusion 2512b constitute the guided part, and the opening 42a and the opening 42b of the outer needle hub 4 and the protrusion 2512a and the protrusion 2512b of the hemostasis valve holder 25 constitute the rotation preventing mechanism.

In addition, the protrusion 2512a and the protrusion 2512b of the hemostasis valve holder 25 are fitted to the opening 42a and the opening 42b of the outer needle hub 4 to constitute the locking part that restricts the relative movement in the axis L1 direction with respect to the outer needle hub 4.

In the fourth modification, the protrusion 2512a and the protrusion 2512b are provided on the claw 251a and the claw 251b provided at symmetrical positions with respect to the axis L1. Instead, a large diameter part having a cylindrical shape may be provided on the rear end side of the diameter expanded part 52 of the hemostasis valve holder 25, and a flange having a triangular cross-sectional shape protruding in the outer diameter direction over the entire circumference may be provided on an outer peripheral surface of the large diameter part. In this case, a part of the flange is fitted to each of the opening 42a and the opening 42b of the outer needle hub 4.

### (Second embodiment)

Hereinafter, an indwelling needle according to a second embodiment of the present invention will be described. The same reference numerals are used for the same components as those of the first embodiment, and the detailed description thereof will be omitted.

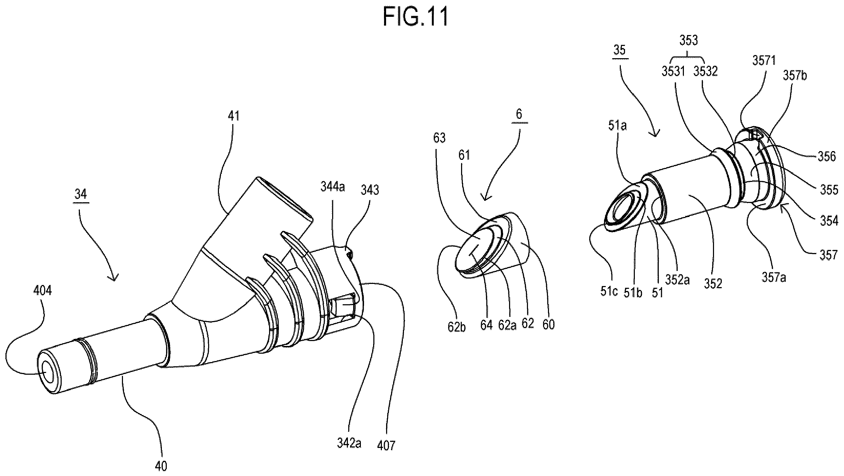

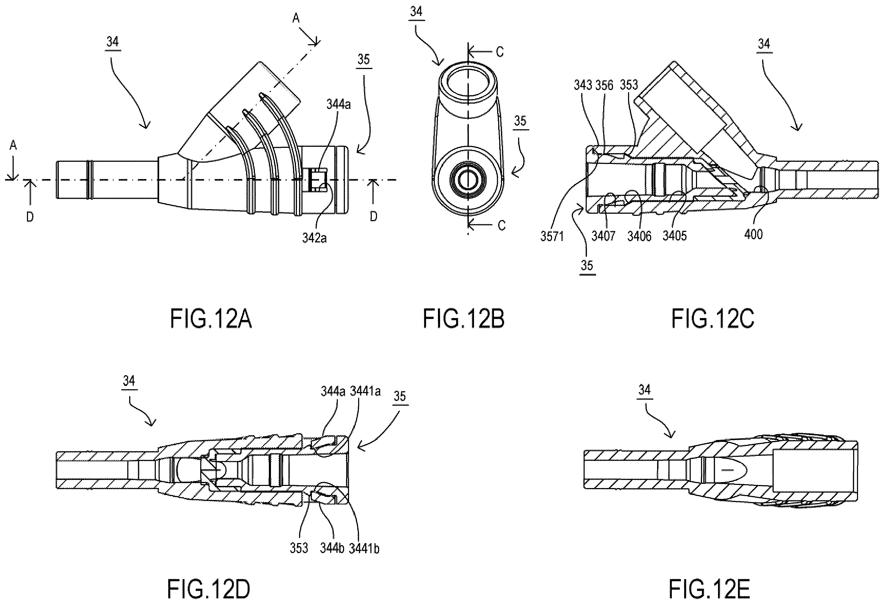

FIG. 11 is an exploded perspective view of an outer needle hub 34, a hemostasis valve holder 35, and the hemostasis valve 6 according to the second embodiment. FIG. 12(A) is a side view illustrating a state in which the hemostasis valve 6 and the hemostasis valve holder 35 are mounted to the outer needle hub 34 according to the second embodiment, and FIG. 12(B) is an end view of the outer needle hub 34 to which the hemostasis valve 6 and the hemostasis valve holder 35 are mounted as viewed from the proximal end side along the axis L1 direction. FIG. 12(C) is a cross-sectional view taken along a direction C-C of the outer needle hub 34 to which the hemostasis valve 6 and the hemostasis valve holder 35 are mounted, and FIG. 12(D) is a cross-sectional view taken along a direction D-D of the outer needle hub 34 to which the hemostasis valve 6 and the hemostasis valve holder 35 are mounted. FIG. 12(E) is a cross-sectional view taken along a direction A-A of the outer needle hub 34 to which the hemostasis valve 6 and the hemostasis valve holder 35 are mounted.

As illustrated in FIG. 11, an opening 342a and an opening 342b having a substantially rectangular shape are opened on the rear end side of the main body 40 of the outer needle hub 34 according to the second embodiment. The opening 342a and the opening 342b are provided at symmetrical positions with respect to the axis L1. In addition, a substantially rectangular parallelepiped protrusion 343 is provided that protrudes in the axis L1 direction from an end surface 407 on the rear end side of the main body 40 of the outer needle hub 34. The protrusion 343 is provided between the opening 342a and the opening 342b, and is provided at a position on the side where the branch part 41 branches with respect to the axis L1. Substantially rectangular claws 344a and 344b are provided so as to be elastically deformable in the opening 342a and the opening 342b of the outer needle hub 34, respectively. The claw 344a and the claw 344b are formed in a shape in which the distal end side is bent toward the inner diameter side, and are connected to the main body 40 on the rear end side.

As illustrated in FIGS. 12(C) and 12(D), a hollow portion 3405 is provided on the rear end side of the hollow portion 400 inside the outer needle hub 34. The inner diameter of the hollow portion 3405 is substantially constant in the axis L1 direction. A hollow portion 3407 having an inner diameter larger than that of the hollow portion 3405 is provided on the rear end side of the hollow portion 3405 via a hollow portion 3406 having a diameter increasing to the outer diameter side. The opening 342a and the opening 342b are opened so as to penetrate the outer needle hub 34 in the outer diameter direction from the hollow portion 3406 to the hollow portion 3407.

As illustrated in FIG. 11, in the hemostasis valve holder 35 according to the second embodiment, a cylindrical medium diameter part 352 having a diameter larger than that of the small diameter part 51 and having a constant diameter in the axis L1 direction is connected to the rear end side of the small diameter part 51 similar to that in the first embodiment via an end surface 352a extending in the direction perpendicular to the axis L1 direction. A cylindrical second medium diameter part 354 having substantially the same diameter as that of the medium diameter part 352 is connected to the rear end side of the medium diameter part 352 via a flange part 353 having, over the entire circumference, a first diameter expanded part 3531 having a diameter increasing toward the rear end side and an end surface 3532 extending in the direction perpendicular to the axis L1 direction. On the rear end side of the second medium diameter part 354, a second diameter expanded part 355 having a diameter increasing toward the rear end side is provided over the entire circumference. A cylindrical large diameter part 356 having a diameter larger than those of the medium diameter part 352 and the second medium diameter part 354, and having a diameter substantially equal to that of the flange part 353 is provided continuously from the second diameter expanded part 355. An annular flange part 357 protruding to the outer diameter side is formed at a rear end part of the large diameter part 356. Provided on the distal end side of the flange part 357 is a recess 3571 that is recessed from a part of an end surface 357a on the distal end side toward the rear end side. The recess 3571 opens from the end surface 357a on the distal end side of the flange part 357 to an outer peripheral surface 357b. The recess 3571 is provided at a position opposite to the side where the inclined first end surface 51a of the small diameter part 51 protrudes to the distal end side with respect to the axis L1.

The hemostasis valve holder 35 to which the hemostasis valve 6 is attached is press-fitted from the opening at the rear end of the outer needle hub 34 to be attached to the outer needle hub 34. When the hemostasis valve holder 35 is press-fitted into the outer needle hub 34, distal end parts 3441a and 3441b of the claws 344a and 344b are pressed to the outer diameter side and elastically deformed by abutting on the first diameter expanded part 3531 of the flange part 353 of the hemostasis valve holder 35. When the hemostasis valve holder 35 is further pushed into the outer needle hub 34 toward the distal end side, the distal end parts 3441a and 3441b of the claws 344a and 344b rub on the first diameter expanded part 3531 of the flange part 353 of the hemostasis valve holder 35. When the hemostasis valve holder 35 is appropriately positioned in the outer needle hub 34, the distal end parts 3441a and 3441b of the claws 344a and 344b climb over the first diameter expanded part 3531 of the flange part 353 of the hemostasis valve holder 35. As illustrated in FIG. 12(D), since the second medium diameter part 354 is provided on the rear end side of the flange part 353 in contact with the end surface 3532, the claw 344a and the claw 344b pressed to the outer diameter side elastically return to the inner diameter side, and the distal end part 3441a and the distal end part 3441b come into abutment with the second medium diameter part 354. At this time, parts of the claws 344a and 344b closer to the rear end than the distal end parts 3441a and 3441b may abut on the second diameter expanded part 355. The first diameter expanded part 3531 of the flange part 353 of the hemostasis valve holder 35 faces and abuts on an inner peripheral surface of the hollow portion 3406 of the outer needle hub 34 inside a part circumferentially sandwiched between the opening 342a and the opening 342b of the outer needle hub 34. At this time, the large diameter part 356 of the hemostasis valve holder 35 faces and abuts on the hollow portion 3407 of the outer needle hub 34. In addition, when the hemostasis valve holder 35 is appropriately positioned in the outer needle hub 34, the protrusion 343 of the outer needle hub is fitted to the recess 3571 of the flange part 357 of the hemostasis valve holder 35.

As described above, the claw 344a and the claw 344b of the outer needle hub 34 are fitted to a recess formed by the flange part 353, the second medium diameter part 354, and the second diameter expanded part 355 of the hemostasis valve holder 35, and the protrusion 343 of the outer needle hub 34 is fitted to the recess 3571 in the axis L1 direction, so that the direction around the axis L1 of the hemostasis valve holder 35 can be guided in a certain direction. Therefore, the direction around the axis L1 of the hemostasis valve 6 attached to the hemostasis valve holder 35 can be defined. Consequently, blood flowing into the outer needle hub 34 from the outer needle 3 can be smoothly moved to the branch part 41, and stagnation in the vicinity of the hemostasis valve 6 can be prevented.

Here, the claw 344a and the claw 344b of the outer needle hub 34 and the flange part 353 of the hemostasis valve holder 35 define relative positions of the hemostasis valve and the hemostasis valve holder in the axis direction with respect to the outer needle hub. The protrusion 343 and the recess 3571 define relative positions of the hemostasis valve and the hemostasis valve holder in the direction around the axis with respect to the outer needle hub. That is, the protrusion 343 constitutes the guiding part and constitutes the locking part, the recess 3571 constitutes the guided part, and both of them constitute the rotation preventing mechanism.

### <Fifth modification>

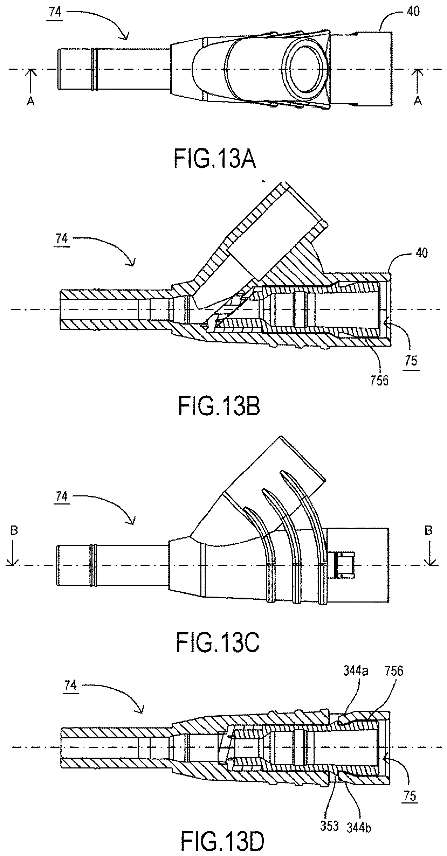

With reference to FIGS. 13(A) to 13(D), an outer needle hub 74 and a hemostasis valve holder 75 according to a fifth modification, which is a modification of the second embodiment, will be described. Since the hemostasis valve 6 and other components of the indwelling needle are similar to those of the above-described embodiments, the detailed description thereof will be omitted. The same reference numerals are used for the same components as those of the first and second embodiments. FIG. 13(A) is a plan view illustrating a state in which the hemostasis valve 6 and the hemostasis valve holder 75 are mounted to the outer needle hub 74 according to the fifth modification, and FIG. 13(B) is a cross-sectional view taken along a direction A-A of the outer needle hub 74 to which the hemostasis valve 6 and the hemostasis valve holder 75 are mounted. FIG. 13(C) is a side view illustrating a state in which the hemostasis valve 6 and the hemostasis valve holder 75 are mounted to the outer needle hub 74 in the indwelling needle according to the fifth modification, and FIG. 13(D) is a cross-sectional view taken along a direction B-B of the outer needle hub 74 to which the hemostasis valve 6 and the hemostasis valve holder 75 are mounted.

As illustrated in FIGS. 13(B) and 13(D), in the fifth modification, no flange part is provided on the rear end side of a large diameter part 756 of the hemostasis valve holder 75, and the large diameter part 756 is provided up to the rear end. In addition, although the outer needle hub 74 according to the fifth modification has substantially the same configuration as that of the second embodiment, the rear end side of the main body 40 is extended along the axis L1 direction. The main body 40 extends to the rear end side beyond the rear end of the appropriately positioned hemostasis valve holder 75. Furthermore, in the fifth modification, the outer needle hub 74 is not provided with the protrusion 343.

Note that the configuration of the hemostasis valve 6 according to the fifth modification is similar to that of the first embodiment and the second embodiment.

As described above, the claws 344a and 344b of the outer needle hub 74 are fitted to the recess formed by the flange part 353, the second medium diameter part 354, and the second diameter expanded part 355 of the hemostasis valve holder 75, so that axial positions of the hemostasis valve 6 and the hemostasis valve holder 75 can be defined. In addition, the hemostasis valve 6 and a peripheral wall part of the outer needle hub 74 can guide the direction around the axis L1 of the hemostasis valve holder 75 in a certain direction.

### <Sixth modification>

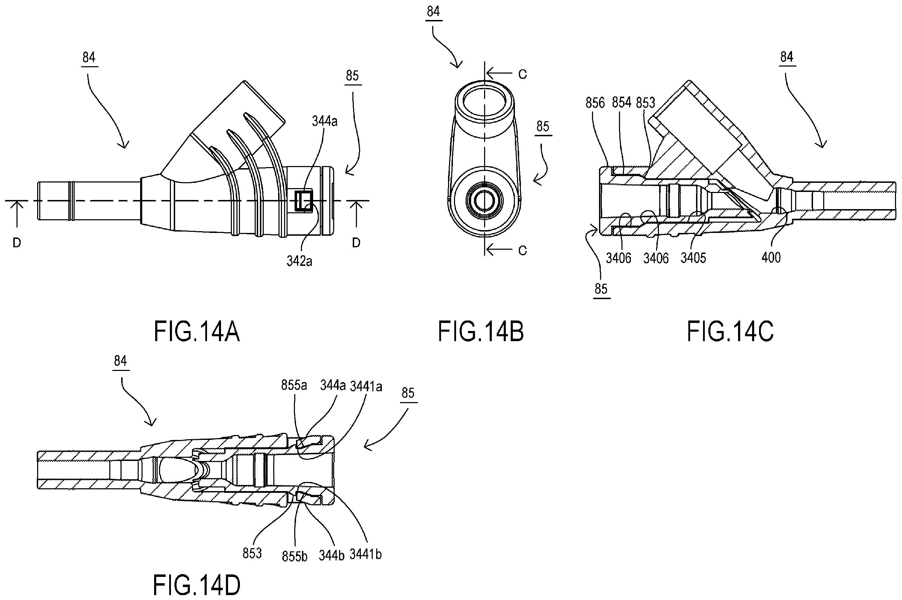

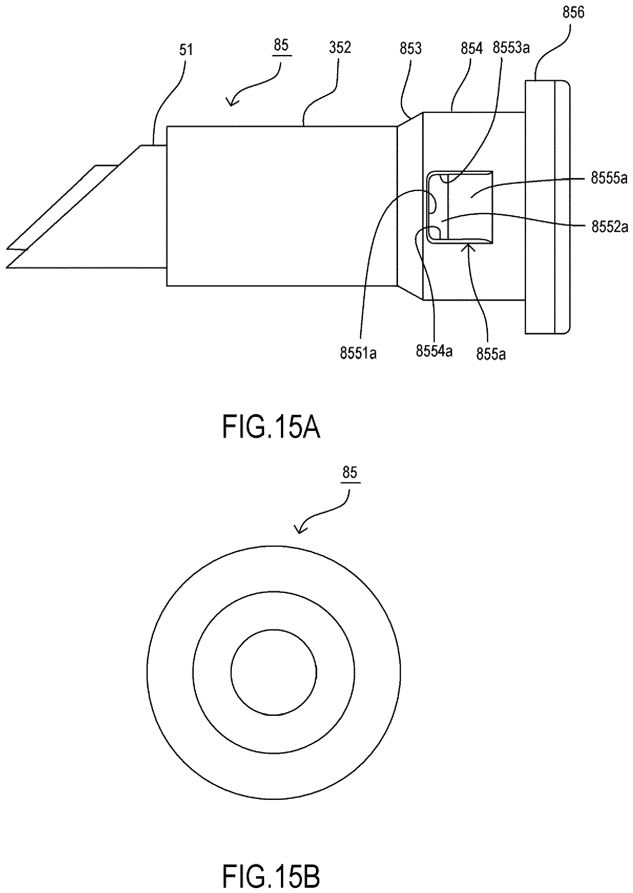

With reference to FIGS. 14 and 15, an outer needle hub 84 and a hemostasis valve holder 85 according to a sixth modification, which is a modification of the second embodiment, will be described. Since the hemostasis valve 6 and other components of the indwelling needle are similar to those of the above-described embodiment, the detailed description thereof will be omitted. The same reference numerals are used for the same components as those of the above-described embodiment.

FIG. 14(A) is a side view of the outer needle hub 84 to which the hemostasis valve holder 85 is mounted, and FIG. 14(B) is an end view of the outer needle hub 84 to which the hemostasis valve holder 85 is mounted as viewed from the proximal end side along the axis L1. FIG. 14(C) is a cross-sectional view taken along a direction A-A of the outer needle hub 84 to which the hemostasis valve holder 85 is mounted, and FIG. 14(D) is a cross-sectional view taken along a direction B-B of the outer needle hub 84 to which the hemostasis valve holder 85 is mounted. FIG. 15(A) is a side view of the hemostasis valve holder 85, and FIG. 15(B) is an end view of the hemostasis valve holder 85 as viewed from the proximal end side.

As illustrated in FIGS. 14(A) and 14(D), similarly to the second embodiment, the opening 342a and the opening 342b having a substantially rectangular shape are opened in the outer needle hub 84 according to the sixth modification, and the substantially rectangular claws 344a and 344b are provided so as to be elastically deformable in the opening 342a and the opening 342b, respectively. Note that the outer needle hub 84 according to the sixth modification is not provided with the protrusion 343.

As illustrated in FIG. 15(A), the hemostasis valve holder 85 is provided with the cylindrical medium diameter part 352 on the rear end side of the small diameter part 51, a diameter expanded part 853 having a diameter increasing toward the rear end side, a large diameter part 854, and a flange part 856 protruding to the outer diameter side. A recess 855a and a recess 855b are provided adjacent to the diameter expanded part 853 on the distal end side of the large diameter part 854. The recess 855a has, on the distal end side, an end surface 8551a extending from an outer surface of the large diameter part 854 to the inner diameter side, a bottom part 8552a provided continuously from an end part on the inner diameter side of the end surface 8551a, a side surface 8553a and a side surface 8554a provided continuously from circumferential opposite end parts of the end surface 8551a, and an inclined surface part 8555a inclined to the outer diameter side from the bottom part 8552a toward the rear end side and provided continuously from the outer surface of the large diameter part. Similarly, the recess 855b has an end surface 8551b, a bottom part 8552b, a side surface 8553b, a side surface 8554b, and an inclined surface part 8555b. The recess 855a and the recess 855b are provided at symmetrical positions with respect to the axis L1.

When the small diameter part 51 of the hemostasis valve holder 85 is press-fitted into the opening on the rear end side of the outer needle hub 84, the distal end parts 3441a and 3441b of the claws 344a and 344b of the outer needle hub 84 are pressed to the outer diameter side and elastically deformed by the diameter expanded part 853 of the hemostasis valve holder 85. When the hemostasis valve holder 85 is further pushed toward the distal end side of the outer needle hub 84 and the claw 344a and the claw 344b reach the recess 855a and the recess 855b, respectively, the claw 344a and the claw 344b elastically return to bend to the inner diameter side, and the distal end parts 3441a and 3441b of the claws 344a and 344b come into abutment with the bottom parts 8552a and 8552b. As a result, the claw 344a and the claw 344b of the outer needle hub 84 are fitted to the recess 855a and the recess 855b of the hemostasis valve holder 85.

As described above, the claw 344a and the claw 344b of the outer needle hub 84 are fitted to the recess 855a and the recess 855b of the hemostasis valve holder 85, so that the direction around the axis L 1 of the hemostasis valve holder 85 can be guided in a certain direction. Therefore, the direction around the axis L1 of the hemostasis valve 6 attached to the hemostasis valve holder 85 can be defined. Consequently, blood flowing into the outer needle hub 84 from the outer needle 3 can be smoothly moved to the branch part 41, and stagnation in the vicinity of the hemostasis valve 6 can be prevented.

Here, the claw 344a and the claw 344b of the outer needle hub 84 constitute the guiding part. The recess 855a and the recess 855b constitute the guided part, and the claw 344a and the claw 344b of the outer needle hub 84 and the recess 855a and the recess 855b of the hemostasis valve holder 85 constitute the rotation preventing mechanism.

In addition, the recess 855a and the recess 855b of the hemostasis valve holder 85 are fitted to the claw 344a and the claw 344b of the outer needle hub 84 to constitute the locking part that restricts the relative movement in the axis L1 direction with respect to the outer needle hub 84.

### <Seventh modification>

Hereinafter, a seventh modification, which is a modification of the first embodiment and the second embodiment, will be described. The same reference numerals are used for the same components as those of the first and second embodiments, and the detailed description thereof will be omitted.

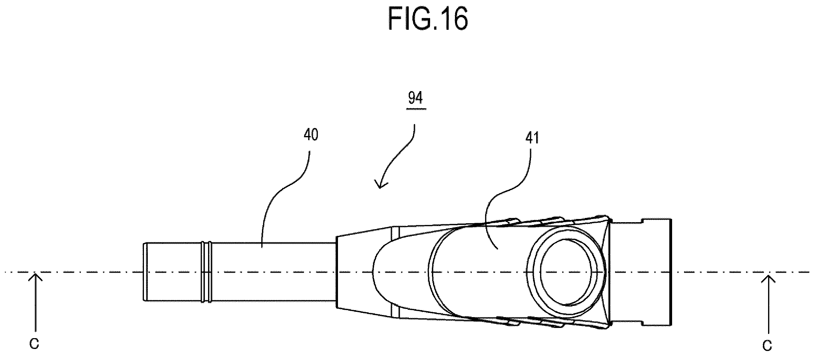

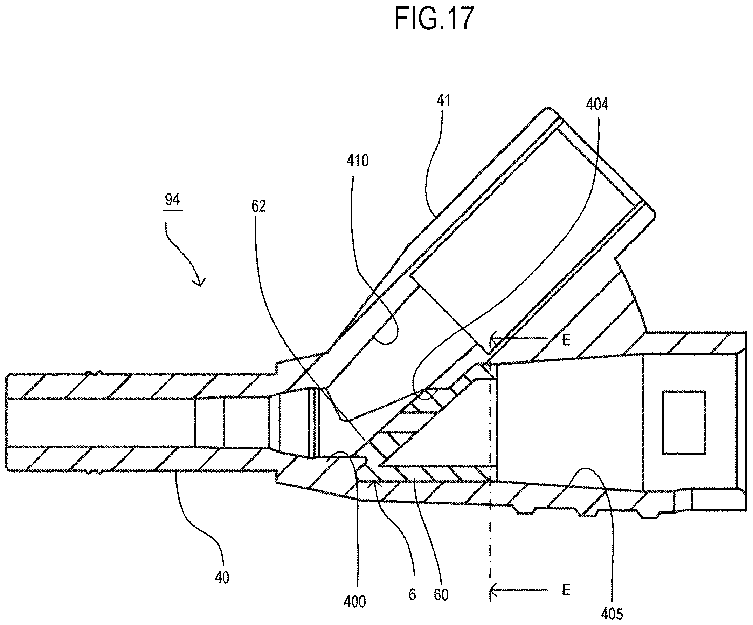

FIG. 16 is a plan view of an outer needle hub 94 to which the hemostasis valve 6 is attached according to the seventh modification. FIG. 17 is a cross-sectional view taken along a direction C-C of the outer needle hub 94 to which the hemostasis valve 6 is attached according to the seventh modification.

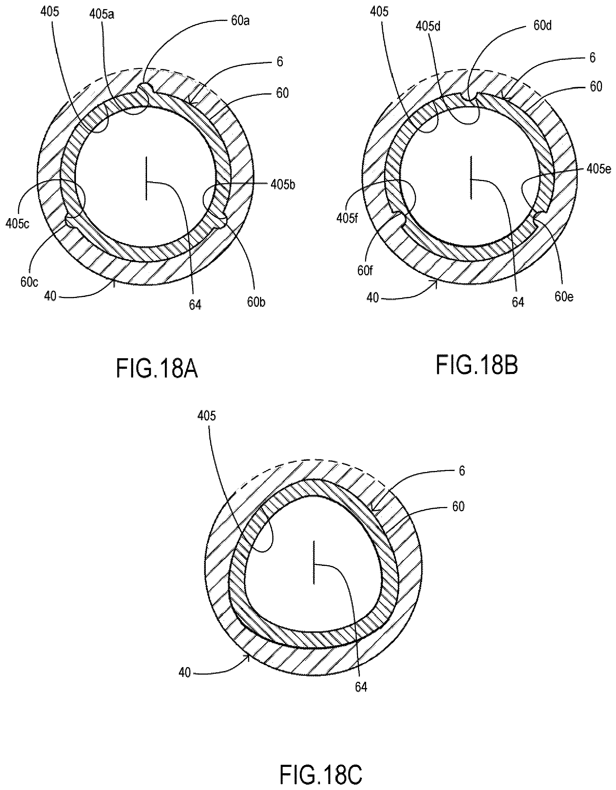

Only the hemostasis valve 6 may be first attached to the outer needle hub 94 as illustrated in FIG. 17, without attaching the hemostasis valve holder 5 to which the hemostasis valve 6 is attached, to the outer needle hub 4 as described in the first and second embodiments. In this case, as illustrated in FIGS. 18(A) to 18(C), the hemostasis valve 6 can be provided with the rotation preventing mechanism. FIGS. 18(A) to 18(C) illustrate the hemostasis valve 6 and the outer needle hub 94 in an E-E cross section of FIG. 17. In this manner, the hemostasis valve 6 can be attached to the outer needle hub 94, and then the hemostasis valve holder 5 can be press-fitted into and mounted to the outer needle hub 94. Alternatively, the hemostasis valve 6 may be made thick without providing the hemostasis valve holder 5.

In FIG. 18(A), ribs 60a, 60b, and 60c in the axis L1 direction are provided at three positions in the circumferential direction of the cylindrical part 60 of the hemostasis valve 6, and grooves 405a, 405b, and 405c in the axis L1 direction are provided at positions corresponding to the ribs 60a, 60b, and 60c on the inner peripheral surface of the hollow portion 405 of the main body 40 of the outer needle hub 94. When the hemostasis valve 6 is press-fitted into the hollow portion 405 of the main body 40 of the outer needle hub 94, the hemostasis valve 6 can be attached to the outer needle hub 94 without rotating the hemostasis valve 6 about the axis L1 by moving the hemostasis valve 6 in the axis L1 direction while fitting the ribs 60a, 60b, and 60c of the hemostasis valve 6 to the grooves 405a, 405b, and 405c on the inner peripheral surface of the hollow portion 405, respectively. That is, the grooves 405a, 405b, and 405c on the inner peripheral surface of the hollow portion 405 of the outer needle hub 94 can guide the direction around the axis L1 of the hemostasis valve 6 in a certain direction. Therefore, the direction around the axis L1 of the hemostasis valve 6 can be defined. Consequently, blood flowing into the outer needle hub 94 from the outer needle 3 can be smoothly moved to the branch part 41, and stagnation in the vicinity of the hemostasis valve 6 can be prevented. Here, the grooves 405a, 405b, and 405c on the inner peripheral surface of the hollow portion 405 of the outer needle hub 94 constitute the guiding part. The ribs 60a, 60b, and 60c of the hemostasis valve 6 constitute the guided part, and the ribs 60a, 60b, and 60c and the grooves 405a, 405b, and 405c on the inner peripheral surface of the hollow portion 405 constitute the rotation preventing mechanism. Note that the positions of the ribs 60a, 60b, and 60c of the hemostasis valve 6 are not limited to the case of being arranged at equal intervals in the circumferential direction of the cylindrical part 60, and may be arranged at different intervals in the circumferential direction of the cylindrical part 60. In this case, the grooves 405a, 405b, and 405c on the inner peripheral surface of the main body 40 of the outer needle hub 94 are also provided at positions corresponding to the ribs 60a, 60b, and 60c of the hemostasis valve 6. In addition, the guiding part may extend to the proximal end of the outer needle hub 94.

In FIG. 18(B), grooves 60d, 60e, and 60f in the axis L1 direction are provided at three positions in the circumferential direction of the cylindrical part 60 of the hemostasis valve 6, and ribs 405d, 405e, and 405f in the axis L1 direction are provided at positions corresponding to the grooves 60d, 60e, and 60f on the inner peripheral surface of the hollow portion 405 of the main body 40 of the outer needle hub 94. When the hemostasis valve 6 is press-fitted into the hollow portion 405 of the main body 40 of the outer needle hub 94, the hemostasis valve 6 can be attached to the outer needle hub 94 without rotating the hemostasis valve 6 about the axis L1 by moving the hemostasis valve 6 in the axis L1 direction while fitting the grooves 60d, 60e, and 60f of the hemostasis valve 6 to the ribs 405d, 405e, and 405f on the inner peripheral surface of the hollow portion 405, respectively. That is, the ribs 405d, 405e, and 405f on the inner peripheral surface of the hollow portion 405 of the outer needle hub 94 can guide the direction around the axis L1 of the hemostasis valve 6 in a certain direction. Therefore, the direction around the axis L1 of the hemostasis valve 6 can be defined. Consequently, blood flowing into the outer needle hub 94 from the outer needle 3 can be smoothly moved to the branch part 41, and stagnation in the vicinity of the hemostasis valve 6 can be prevented. Here, the ribs 405d, 405e, and 405f on the inner peripheral surface of the hollow portion 405 of the outer needle hub 94 constitute the guiding part. The grooves 60d, 60e, and 60f of the hemostasis valve 6 constitute the guided part, and the grooves 60d, 60e, and 60f and the ribs 405d, 405e, and 405f on the inner peripheral surface of the hollow portion 405 constitute the rotation preventing mechanism. Note that the positions of the grooves 60d, 60e, and 60f of the hemostasis valve 6 are not limited to the case of being arranged at equal intervals in the circumferential direction of the cylindrical part 60, and may be arranged at different intervals in the circumferential direction of the cylindrical part 60. In this case, the ribs 405d, 405e, and 405f on the inner peripheral surface of the main body 40 of the outer needle hub 94 are also provided at positions corresponding to the grooves 60d, 60e, and 60f of the hemostasis valve 6. In addition, the guiding part may extend to the proximal end of the outer needle hub 94.

In FIG. 18(C), the cylindrical part 60 of the hemostasis valve 6 has a triangular tube shape with rounded corners, and the hollow portion 405 of the main body 40 of the outer needle hub 94 has a triangular prism shape with rounded corners. The hemostasis valve 6 having such a shape is configured such that a portion where a distance from the axis L1 to an outer peripheral surface of the cylindrical part 60 is long and a portion where the distance is short appear at different positions in the circumferential direction. When the hemostasis valve 6 is press-fitted into the hollow portion 405 of the main body 40 of the outer needle hub 94, the hemostasis valve 6 can be attached to the outer needle hub 94 without rotating the hemostasis valve 6 about the axis L1 by moving the hemostasis valve 6 in the axis L1 direction while matching the outer shape of the cylindrical part 60 of the hemostasis valve 6 with the inner peripheral shape of the hollow portion 405. That is, the inner peripheral shape of the hollow portion 405 of the outer needle hub 94 can guide the direction around the axis L1 of the hemostasis valve 6 in a certain direction. Therefore, the direction around the axis L1 of the hemostasis valve 6 can be defined. Consequently, blood flowing into the outer needle hub 94 from the outer needle 3 can be smoothly moved to the branch part 41, and stagnation in the vicinity of the hemostasis valve 6 can be prevented. Here, the inner peripheral shape of the hollow portion 405 constitutes the guiding part. The outer shape of the cylindrical part 60 of the hemostasis valve 6 constitutes the guided part, and the outer shape of the cylindrical part 60 and the inner peripheral shape of the hollow portion 405 constitute the rotation preventing mechanism. Note that the shape of the cylindrical part 60 of the hemostasis valve 6 is not limited to the above-described shape, and may be a shape in which the portions with a long distance and a short distance from the axis L1 to the outer peripheral surface of the cylindrical part 60 appear at unequal intervals in the circumferential direction, or may be a shape in which the length of the distance from the axis L1 to the outer peripheral surface of the cylindrical part 60 is different in the circumferential direction. Note that the guided part illustrated in FIGS. 18(A) to 18(C) may be provided in the hemostasis valve holder.

### <Eighth modification>

Hereinafter, an eighth modification, which is a modification of the first embodiment and the second embodiment, will be described. The same reference numerals are used for the same components as those of the first and second embodiments, and the detailed description thereof will be omitted.

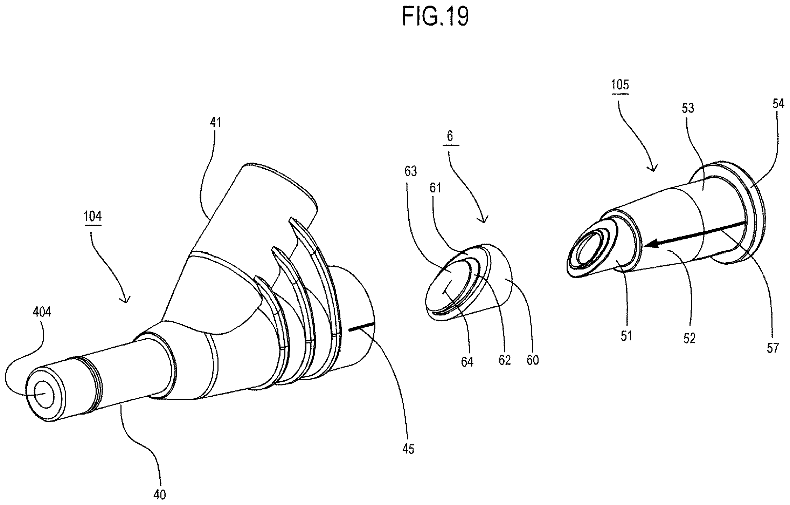

FIG. 19 is an exploded perspective view of an outer needle hub 104, the hemostasis valve 6, and a hemostasis valve holder 105 according to the eighth modification.

As illustrated in FIG. 19, an indication 45 for alignment of a relative positional relationship in the rotation direction about the axis L1 is given to an outer peripheral surface on the rear end side of the main body 40 of the outer needle hub 104. An indication for alignment of the relative positional relationship in the rotation direction about the axis L1 is also given to corresponding positions on outer peripheral surfaces of the diameter expanded part 52 and the large diameter part 53 of the hemostasis valve holder 105. To align the direction of the indication 45 of the outer needle hub 104 and the direction of an indication 57 of the hemostasis valve holder 105 with each other when attaching the hemostasis valve holder 105, to which the hemostasis valve 6 is attached, to the outer needle hub 104, the hemostasis valve holder 105 is press-fitted from the opening on the rear end side of the outer needle hub 104 while maintaining the posture of the hemostasis valve holder 105 with respect to the outer needle hub 104 by reference to the indications 45 and 57. This makes it possible to prevent the rotation of the hemostasis valve 6 and the hemostasis valve holder 105 about the axis L 1. That is, the indication 45 of the outer needle hub 104 can guide the direction around the axis L1 of the hemostasis valve holder 105 in a certain direction. Therefore, the direction around the axis L1 of the hemostasis valve 6 can be defined. Consequently, blood flowing into the outer needle hub 104 from the outer needle 3 can be smoothly moved to the branch part 41, and stagnation in the vicinity of the hemostasis valve 6 can be prevented. Here, the indication 45 of the outer needle hub 104 constitutes the guiding part. The indication 57 of the hemostasis valve holder 105 constitutes the guided part. The indication 45 of the outer needle hub 104 and the indication 57 of the hemostasis valve holder 105 constitute the rotation preventing mechanism. The indication for alignment with the indication of the outer needle hub 104 may be given to the outer peripheral surface of the cylindrical part 60 of the hemostasis valve 6, or may be given to the outer peripheral surfaces of both the hemostasis valve 6 and the hemostasis valve holder 105.

In addition, as the contents of the indications, characters, symbols, figures, and the like can be used, and any form may be employed as long as an operator can recognize the contents. Furthermore, the indications are not limited to being two-dimensionally expressed by printing or the like, and may be three-dimensionally expressed.

### (Third embodiment)

Hereinafter, an indwelling needle according to a third embodiment of the present invention will be described. The same reference numerals are used for the same components as those of the first and second embodiments, and the description thereof will be omitted.

Since the inner needle 2 and the inner needle hub 21 of the indwelling needle are similar to those of the first embodiment, the description thereof will be omitted.

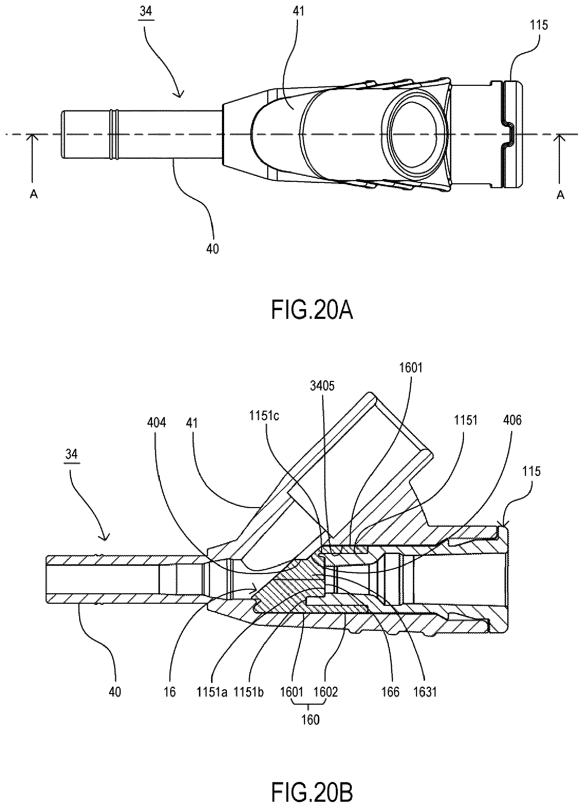

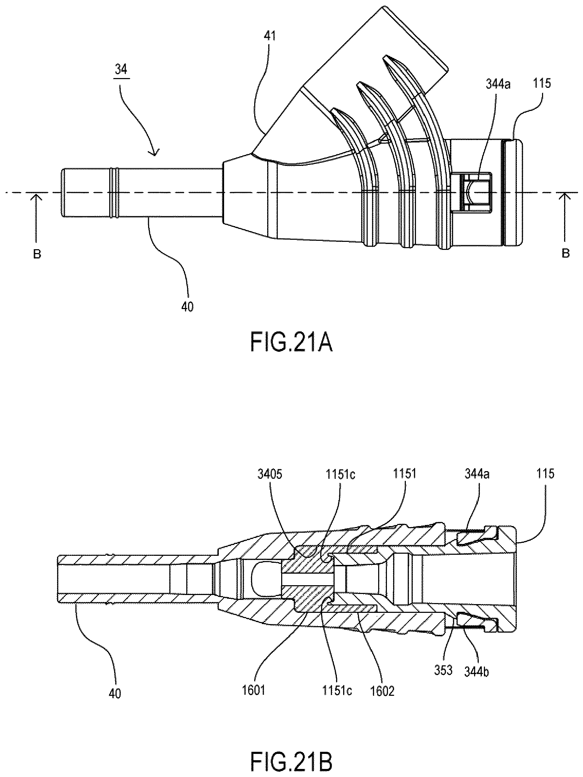

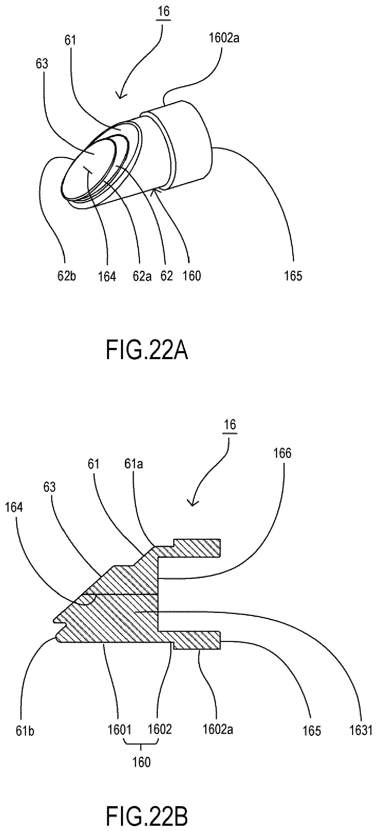

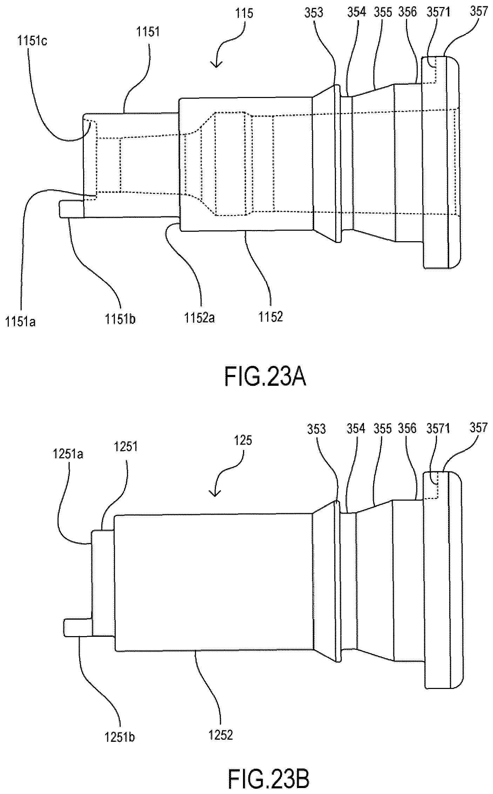

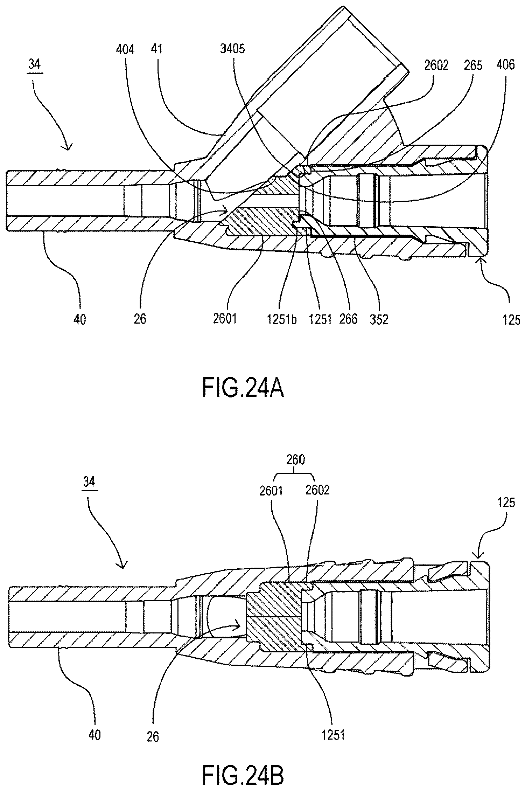

The outer needle hub 34 according to the third embodiment is similar to that of the second embodiment illustrated in FIG. 11, whereas a hemostasis valve 16 and a hemostasis valve holder 115 are different from those of the first and second embodiments. FIG. 20(A) illustrates a plan view of the outer needle hub 34 to which the hemostasis valve 16 and the hemostasis valve holder 115 of the third embodiment are mounted, and FIG. 20(B) illustrates an A-A cross-sectional view of the same. FIG. 21(A) illustrates a side view of the outer needle hub 34 to which the hemostasis valve 16 and the hemostasis valve holder 115 of the third embodiment are mounted, and FIG. 21(B) illustrates a B-B cross-sectional view of the same. FIG. 22(A) illustrates a perspective view of the hemostasis valve 16 of the third embodiment, and FIG. 22(B) illustrates an A-A cross-sectional view of the same. FIG. 23(A) illustrates a side view of the hemostasis valve holder 115 of the third embodiment, and FIG. 23(B) illustrates a side view of a hemostasis valve holder 125 of a reference example. FIG. 24(A) illustrates an A-A cross-sectional view of the outer needle hub 34 to which a hemostasis valve 26 and the hemostasis valve holder 125 of the reference example are mounted, and FIG. 24(B) illustrates a B-B cross-sectional view of the same.

As illustrated in FIG. 22(A), the hemostasis valve 16 includes the first end surface 61, the step part 62, and the second end surface 63 on the distal end side similarly to the first and second embodiments. A slit 164 of the hemostasis valve 16 is provided in a direction perpendicular to a plane including the axis L1 and the axis L2, and the direction of the slit 64 is perpendicular to the direction of the slit 164 of the hemostasis valve 6 of the first embodiment. Since the central proximal end surface 66 of the hemostasis valve 6 according to the first and second embodiments is formed in parallel with the first end surface 61 and the second end surface 63 to be inclined with respect to the axis L1 direction, the soft solid part 631 sandwiched between the first end surface 61 and the second end surface 63, and the central proximal end surface 66 is thin. On the other hand, as illustrated in FIG. 22(B), a central proximal end surface 166 of the hemostasis valve 16 is formed to be perpendicular to the axis L1 direction instead of being parallel to the first end surface 61 and the second end surface 63. Therefore, in the hemostasis valve 16, a soft solid part 1631 sandwiched between the first and second end surfaces 61 and 63 and the central proximal end surface 166 is formed thick. Here, in the first end surface 61 of the hemostasis valve 16, a part connected to a cylindrical part 160 on the most proximal end side is referred to as an end point 61a of the first end surface 61, and a part at a symmetrical position to the end point of the first end surface 61 with respect to the axis L1 and connected to the cylindrical part 160 on the most distal end side is referred to as a starting point 61b of the first end surface 61. In the hemostasis valve 16, the cylindrical part 160 includes a first cylindrical part 1601 having the solid part 1631 on the inner diameter side, and a second cylindrical part 1602 extending from the first cylindrical part 1601 to the proximal end side along the axis L1 direction and having a hollow portion on the inner diameter side. Here, the second cylindrical part 1602 corresponds to a cylindrical part of the present invention. In addition, in the hemostasis valve 16, the length in the axis L1 direction of a portion of the cylindrical part 160 (here, the portion coincides with the second cylindrical part 1602) extending from the end point 61a of the first end surface 61 to the proximal end side is set to be longer than the length in the axis L1 direction of a portion of the cylindrical part 60 according to the first and second embodiments extending from the end point 61a of the first end surface 61 to the proximal end side. The portion extending from the end point 61a of the first end surface 61 to the proximal end side has a length of 1 mm or more, preferably 2 mm or more, in the axis L1 direction from the end point 61a of the first end surface 61. In addition, in the second cylindrical part 1602 of the hemostasis valve 16, a thick part 1602a having a large thickness in the radial direction is provided on the proximal end side along the axis L1 direction with respect to the end point of the first end surface 61. As the thick part 1602a, a large diameter part having a rectangular cross section and protruding to the outer diameter side is formed over the entire circumference around the axis L1. The thick part 1602a is provided on the rear end side of the second cylindrical part 1602. The outer diameter of the thick part 1602a is set to be larger than the inner diameter of a part of the hollow portion 3405 of the outer needle hub 34 facing the thick part 1602a when the hemostasis valve 16 is appropriately positioned in the outer needle hub 34. In addition, the thickness of the thick part 1602a in the radial direction is set to be larger than an interval between a small diameter part 1151 of the hemostasis valve holder 115 that supports the second cylindrical part 1602 from the inner diameter side and the inner peripheral surface of the hollow portion 3405 of the outer needle hub 34 facing the small diameter part 1151 via the second cylindrical part 1602 when the hemostasis valve 16 and the hemostasis valve holder 115 are appropriately positioned in the outer needle hub 34. The cross-sectional shape of the thick part 1602a is not limited to the configuration in which the thickness in the axis L1 direction is constant, and an appropriate shape can be adopted. A region of the second cylindrical part 1602 where the thick part 1602a is formed is not limited to a region on the rear end side, and the thick part 1602a can be provided in a part or all of an appropriate region of a region from the end point 61a of the first end surface 61 to an end surface 165 on the rear end side. In addition, the region of the second cylindrical part 1602 where the thick part 1602a is formed is not limited to the entire circumference around the axis L1, and the thick part 1602a can be provided in a region from the end point 61a of the first end surface 61 to the end surface 165 on the rear end side, that is, in a part in the circumferential direction including a region in the direction in which the branch part 41 branches.

In accordance with the configuration of the soft solid part 1631 and the central proximal end surface 166 of the hemostasis valve 16 as described above, the small diameter part 1151 of the hemostasis valve holder 115 has a cylindrical shape, and a substantially annular end surface 1151a is formed at an end part on the distal end side of the small diameter part 1151 in the direction perpendicular to the axis L1 direction as illustrated in FIG. 23(A). A protrusion 1151b protruding in the axis L1 direction is formed on the end surface 1151a. The protrusion 1151b is provided on the side opposite to the recess 3571 with respect to the axis L1. The shape of the protrusion 1151b is not limited, but its distal end part desirably has a rounded shape. The end surface 1151a is provided with an annular protrusion 1151c protruding in the axis L1 direction similarly to the protrusion 1151b. The annular protrusion 1151c is formed over the entire circumference on an outer peripheral edge of the end surface 1151a. In the hemostasis valve holder 115, the shape of the end part on the distal end side of the small diameter part 1151 is different from that of the hemostasis valve holder 35, and the length in the axis L 1 direction of the small diameter part 1151 is set to be longer than that of the small diameter part 51 of the hemostasis valve holder 35. Here, the small diameter part 1151 corresponds to an attachment-target part of the present invention. The hemostasis valve holder 115 is configured similarly to the hemostasis valve holder 35 except that the length in the axis L1 direction of a large diameter part 1152 on the proximal end side from an end surface 1152a provided on the proximal end side of the small diameter part 1151 of the hemostasis valve holder 115 is shorter than that of the hemostasis valve holder 35.

The outer dimension of the small diameter part 1151 of the hemostasis valve holder 115 is set to be slightly smaller than the inner diameter dimension of the cylindrical part 160 of the hemostasis valve 16. When the small diameter part 51 of the hemostasis valve holder 115 is fitted into the cylindrical part 160 of the hemostasis valve 16 from the distal end side, the cylindrical part 160 of the hemostasis valve 16 slightly increases in diameter to the outer diameter side, but tries to decrease in diameter to be elastically deformed. The elastic deformation produces a frictional force between the inner peripheral surface of the cylindrical part 160 of the hemostasis valve 16 and the outer peripheral surface of the small diameter part 1151 of the hemostasis valve holder 115. When the hemostasis valve holder 115 is fitted into the hemostasis valve 16 until the end surface 165 on the rear end side of the cylindrical part 160 of the hemostasis valve 16 abuts on the end surface 1151a as the distal end surface of the hemostasis valve holder 115, the end surface 1151a of the hemostasis valve holder 115 abuts on the central proximal end surface 166 of the hemostasis valve 16, and the protrusion 1151b of the hemostasis valve holder 115 presses the central proximal end surface 166. When the hemostasis valve 16 is appropriately mounted to the hemostasis valve holder 115, the protrusion 1151b coincides with a direction around the axis L1 in which the first end surface 61 and the second end surface 63 of the hemostasis valve 16 are located on the most distal end side. Therefore, when the hemostasis valve 16 is mounted to the hemostasis valve holder 115, the protrusion 1151b functions as a guide in mounting the hemostasis valve 16 in a predetermined direction around the axis L1. In addition, since the protrusion 1151b presses and slightly deforms the central proximal end surface 166 of the hemostasis valve 16, the protrusion 1151b constitutes the rotation preventing mechanism that restricts the relative rotation about the axis L1 between the hemostasis valve holder 115 and the hemostasis valve 16. Furthermore, since the annular protrusion 1151c also presses and slightly deforms the central proximal end surface 166 of the hemostasis valve 16, it is possible to prevent displacement between the hemostasis valve holder 115 and the hemostasis valve 16 and to suppress generation of a gap that causes liquid leakage between the hemostasis valve 16 and the outer needle hub 34.

In addition, as illustrated in FIG. 22(B), the central proximal end surface 166 of the hemostasis valve 16 is located in the vicinity of the proximal end side of the end point 61a of the first end surface 61 along the axis L1 direction, and the end surface 1151a of the hemostasis valve holder 115 is fitted up to this position. Therefore, when the hemostasis valve 16 is appropriately attached to the hemostasis valve holder 115, the small diameter part 1151 of the hemostasis valve holder 115 is arranged on the inner diameter side of the second cylindrical part 1602 of the hemostasis valve 16, and the second cylindrical part 1602 is supported by the small diameter part 1151 from the inner diameter side. As described above, when the hemostasis valve 16 and the hemostasis valve holder 115 are appropriately positioned in the outer needle hub 34, the thickness of the thick part 1602a in the radial direction is set to be larger than the interval between the small diameter part 1151 of the hemostasis valve holder 115 that supports the second cylindrical part 1602 from the inner diameter side and the inner peripheral surface of the hollow portion 3405 of the outer needle hub 34 facing the small diameter part 1151 via the second cylindrical part 1602. Therefore, when the hemostasis valve holder 115 to which the hemostasis valve 16 is attached is appropriately positioned with respect to the outer needle hub 34 as illustrated in FIGS. 20(B) and 21(B), the thick part 1602a is compressed between the hemostasis valve holder 115 and the inner peripheral surface of the hollow portion 3405 of the outer needle hub 34. When the thick part 1602a of the hemostasis valve 16 is compressed in the radial direction, the thick part 1602a comes into pressure contact with the inner peripheral surface of the hollow portion 3405 of the outer needle hub 34 by an elastic force, so that a gap between the cylindrical part 160 of the hemostasis valve 16 and the hollow portion 3405 of the outer needle hub 34 is sealed. Therefore, even if a gap is generated between the step part 62 and the first end surface 61 of the hemostasis valve 16, and the opening 404 and the step part 406 of the outer needle hub 34, and also between the cylindrical part 160 of the hemostasis valve 16 and the inner peripheral surface of the hollow portion 3405 of the outer needle hub 34 due to backlash or displacement of the components, deformation of the hemostasis valve 16, or the like, it is possible to suppress leakage of a fluid flowing through the hollow portion 400 and the hollow portion 410, to the proximal end side beyond the thick part 1602a of the hemostasis valve 16.

FIG. 23(B) illustrates a side view of the hemostasis valve holder 125 of the reference example. FIG. 24(A) is an A-A cross-sectional view (see FIG. 20(A)) illustrating a state in which the hemostasis valve holder 125 to which the hemostasis valve 26 of the reference example is attached is appropriately mounted to the outer needle hub 34, and FIG. 24(B) is a B-B cross-sectional view of the same (see FIG. 21(A)). The same reference numerals are used for the same components as those of the first to third embodiments.

Similarly to the hemostasis valve 16, a central proximal end surface 266 of the hemostasis valve 26 is formed in the direction perpendicular to the axis L1 direction, and an end surface 1251a on the distal end side of a small diameter part 1251 of the hemostasis valve holder 125 is also correspondingly formed in the direction perpendicular to the axis L1 direction. In addition, a protrusion 1251b protruding in the axis L1 direction is formed on the end surface 1251a of the hemostasis valve holder 125. The shape and function of the protrusion 1251b are similar to those of the protrusion 1151b. In the hemostasis valve 26, the length in the axis direction of a first cylindrical part 2601 of a cylindrical part 260 is the same as the length in the axis L1 direction of the first cylindrical part 1601 of the hemostasis valve 16, whereas the length in the axis L1 direction of a second cylindrical part 2602 is set to be shorter than the length in the axis L1 direction of the second cylindrical part 1602 of the hemostasis valve 16. Correspondingly, the length in the axis L1 direction of the small diameter part 1251 of the hemostasis valve holder 125 is set to be shorter than that of the small diameter part 1151 of the hemostasis valve holder 115, and the length in the axis direction of a large diameter part 1252 of the hemostasis valve holder 125 is set to be longer than that of the large diameter part 1152 of the hemostasis valve holder 115.

Suppose that a gap is generated between the step part 62 and the first end surface 61 of the hemostasis valve 26, and the opening 404 and the step part 406 of the outer needle hub 34, and also between the cylindrical part 260 of the hemostasis valve 26 and the inner peripheral surface of the hollow portion 3405 of the outer needle hub 34 due to backlash or displacement of the components, deformation of the hemostasis valve 26, or the like when the length from the end point 61a of the first end surface 61 to an end surface 265 on the rear end side in the cylindrical part 260 is short, for example, less than 1 mm as in the hemostasis valve 26 of the reference example. In this case, there is a possibility that the fluid flowing through the hollow portion 400 and the hollow portion 410 leaks out to the proximal end side beyond the second cylindrical part 2602 of the hemostasis valve 26.

The hemostasis valve 16 and the hemostasis valve holder 115 of the third embodiment solve the problem of the hemostasis valve 26 of the reference example by setting the length of the second cylindrical part 1602 extending from the end point 61a of the first end surface 61 to the proximal end side to 1 mm or more, preferably 2 mm or more.

### <Tenth modification>

In the third embodiment, the central proximal end surface 166 is formed in the direction perpendicular to the axis L1 direction as the hemostasis valve 16. However, the third embodiment can also be applied to a case where the central proximal end surface 66 is inclined with respect to the axis L1 direction and formed in parallel with the first end surface 61 and the second end surface 63 as in the hemostasis valve 6 of the first and second embodiments and the modifications thereof. In the hemostasis valve 6, the entire portion on the inner diameter side of the cylindrical part 60 extending from the solid part 631 to the proximal end side in the axis L1 direction is hollow. In the cylindrical part 60, a part on the distal end side from the end point 61a of the first end surface 61 is referred to as a first cylindrical part, and a part extending from the end point 61a to the proximal end side is referred to as a second cylindrical part. The length of the second cylindrical part of the hemostasis valve 6 defined as described above is set to 1 mm or more, preferably 2 mm or more, and a thick part is provided in the second cylindrical part. In this case, the length in the axis L1 direction of the small diameter part 51 of each of the hemostasis valve holders 5, 15, 25, 35, 75, 85, and 105 is also set to correspond to the length in the axis L1 direction of the second cylindrical part. As a result, even if a gap is generated between the step part 62 and the first end surface 61 of the hemostasis valve 6, and the opening 404 and the step part 406 of the outer needle hub 34, and also between the cylindrical part 60 of the hemostasis valve 6 and the inner peripheral surface of the hollow portion 3405 of the outer needle hub 34 due to backlash or displacement of the components, deformation of the hemostasis valve 6, or the like, it is possible to suppress leakage of the fluid flowing through the hollow portion 400 and the hollow portion 410, to the proximal end side beyond the thick part 1602a of the hemostasis valve 6. Here, the second cylindrical part defined as described above corresponds to the cylindrical part of the present invention.

The embodiments and modifications disclosed above can be combined with each other.

### Reference Signs List

- 1: indwelling needle
- 2: inner needle
- 3: outer needle
- 4, 34, 74, 84: outer needle hub
- 5, 15, 25, 35, 75, 85: hemostasis valve holder
- 6: hemostasis valve
- 40: main body
- 41: branch part
- 42a, 42b, 342a, 342b: opening
- 43: slit
- 44: indication
- 51: small diameter part
- 55a, 55b: protrusion
- 56: rib
- 57: indication
- 60a, 60b, 60c: rib
- 60d, 60e, 60f: groove
- 344a, 344b.: claw
- 344.: protrusion
- 405a, 405b, 405c: groove
- 405d, 405e, 405f: rib

## Claims

1. An indwelling needle comprising:
an inner needle that punctures a living body;
an outer needle that is penetrated by the inner needle and is inserted into the living body together with the inner needle as the inner needle punctures the living body;
an outer needle hub including a main body that is arranged along an axis of the inner needle and a branch part that branches from the main body, the outer needle hub having a passage communicating with an inside of the outer needle;
an elastic member that is disposed inside the outer needle hub, is penetrated by the inner needle, and has a soft solid part that blocks a fluid flow to a proximal end side of the main body in a case where the inner needle is removed; and
a holder that is attached to a proximal end of the elastic member, wherein
the elastic member has an end surface that forms at least a part of the passage bringing the inside of the outer needle and the branch part into communication, and that is inclined with respect to a direction of the axis, and
the outer needle hub has a guiding part that is engaged with the elastic member or the holder to guide a direction around the axis of the elastic member in a certain direction.

2. An indwelling needle comprising:
an inner needle that punctures a living body;
an outer needle that is penetrated by the inner needle and is inserted into the living body together with the inner needle as the inner needle punctures the living body;
an outer needle hub including a main body that is arranged along an axis of the inner needle and a branch part that branches from the main body, the outer needle hub having a passage communicating with an inside of the outer needle; and
an elastic member that is disposed inside the outer needle hub, is penetrated by the inner needle, and has a soft solid part that blocks a fluid flow to a proximal end side of the main body in a case where the inner needle is removed, wherein
the elastic member has an end surface that forms at least a part of the passage bringing the inside of the outer needle and the branch part into communication, and that is inclined with respect to a direction of the axis, and
the outer needle hub has a guiding part that is engaged with the elastic member to guide a direction around the axis of the elastic member in a certain direction.

3. An indwelling needle comprising:
an inner needle that punctures a living body;
an outer needle that is penetrated by the inner needle and is inserted into the living body together with the inner needle as the inner needle punctures the living body;
an outer needle hub including a main body that is arranged along an axis of the inner needle and a branch part that branches from the main body, the outer needle hub having a passage communicating with an inside of the outer needle; and
an elastic member that is disposed inside the outer needle hub, is penetrated by the inner needle, and has a soft solid part that blocks a fluid flow to a proximal end side of the main body in a case where the inner needle is removed, wherein
the elastic member has a central part having an end surface that forms at least a part of the passage bringing the inside of the outer needle and the branch part into communication, and that is inclined with respect to a direction of the axis, and an outer peripheral part, and
a peripheral wall part having a proximal end surface facing a distal end surface of the outer peripheral part is formed on an inner surface of the outer needle hub, and at least a part of the central part is located inside the peripheral wall part.

4. The indwelling needle according to claim 1, wherein
the holder includes an attachment-target part to which the elastic member is attached, and
the elastic member includes, on a proximal end side of the solid part, a cylindrical part whose inner diameter side is supported by the attachment-target part, the cylindrical part having a radial thickness larger than an interval between the attachment-target part and a surface of the outer needle hub radially facing the attachment-target part in a state before being mounted to the outer needle hub.

5. The indwelling needle according to claim 2 or 3, comprising
a holder that is attached to a proximal end of the elastic member and includes an attachment-target part to which the elastic member is attached, wherein
the elastic member includes, on a proximal end side of the solid part, a cylindrical part whose inner diameter side is supported by the attachment-target part, the cylindrical part having a radial thickness larger than an interval between the attachment-target part and a surface of the outer needle hub radially facing the attachment-target part in a state before being mounted to the outer needle hub.
